# EUROPEAN PATENT APPLICATION

(11) **EP 2 772 257 A1**
(43) Date of publication of application: **03.09.2014**
(21) Application number: 14150490.2
(22) Date of filing: 29.11.2006
(51) Int. Cl.: A61K 31/496, A61K 47/26, A61K 47/12, A61K 47/36, A61K 47/32, A61P 35/00, A61P 35/02, A61K 9/48

(54) **Formulations of quinolinones**

(30) Priority: 29.11.2005 US 740577 P; 01.12.2005 US 741317 P
(62) Divisional of application: 06838590.5
(71) Applicant: Novartis AG, 4056 Basel (CH)
(72) Inventor: Chou, Joyce, Emeryville, CA California 94608-2916 (US); Okhamafe, Augustus, Emeryville, CA California 94608-2916 (US); Frech, Patricia, Emeryville, CA California 94608-2916 (US); Gullapalli, Rampurma, Emeryville, CA California 94608-2916 (US)
(74) Representative: Rudge, Sewkian

(57) **Abstract**

A pharmaceutical formulation, comprising: a compound of formula I, a tautomer of the compound, a salt of the compound, a salt of the tautomer, or a mixture thereof, 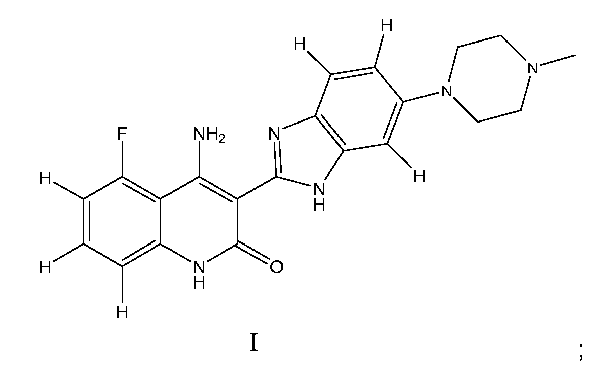 and
at least one ingredient selected from the group consisting of (i) cellulose; (ii) silicon dioxide; (iii) magnesium stearate; and (iv) an ingredient selected from crospovidone, starch, or lactose.

## Description

### FIELD OF THE INVENTION

This invention pertains generally to formulations of quinolinone compounds. More specifically, the invention described herein pertains to solid dosage formulations comprising pharmaceutically acceptable salts such as lactic acid salts of 4-amino-5-fluoro-3-[6-(4-methyl-piperazin-1-yl)-1H-benzimidazol-2-yl]-1 H-quinolin-2-one, and to methods for preparing and using such formulations.

### BACKGROUND OF THE INVENTION

A variety of chemical compounds and compositions have been reported as having activity against one or more vascular endothelial growth factor receptor tyrosine kinase (VEGF-RTK). Examples include quinoline derivatives such as described in WO 98/13350, aminonicotinamide derivatives (see, e.g. WO 01/55114), antisense compounds (see, e.g. WO 01/52904), peptidomimetics (see, e.g. WO 01/52875), quinazoline derivatives (see, e.g. U.S. Patent No. 6,258,951) monoclonal antibodies (see, e.g. EP 1 086 705 A1), various 5,10,15,20-tetraaryl-porphyrins and 5,10,15-triaryl-corroles (see, e.g. WO 00/27379), heterocyclic alkanesulfonic and alkane carboxylic acid derivatives (see, e.g. DE19841985), oxindolylquinazoline derivatives (see, e.g. WO 99/10349), 1,4-diazaanthracine derivatives (see, e.g. U.S. Patent No. 5,763,441), and cinnoline derivatives (see, e.g. WO 97/34876), and various indazole compounds (see, e.g. WO 01/02369 and WO 01/53268).

The synthesis of 4-hydroxy quinolone and 4-hydroxy quinoline derivatives is disclosed in a number of references. For example, Ukrainets et al. have disclosed the synthesis of 3-(benzimidazol-2-yl)-4-hydroxy-2-oxo-1,2-dihydroquinoline. Ukrainets, I. et al., Tetrahedron Lett. 42, 7747-7748 (1995); Ukrainets, I. et al., Khimiya Geterotsiklicheskikh Soedinii, 2, 239-241(1992). Ukrainets has also disclosed the synthesis, anticonvulsive and antithyroid activity of other 4-hydroxy quinolones and thio analogs such as 1 H-2-oxo-3-(2-benzimidazolyl)-4-hydroxyquinoline. Ukrainets, I. et al., Khimiya Geterotsiklicheskikh Soedinii, 1, 105-108 (1993); Ukrainets, I. et al., Khimiya Geterotsiklicheskikh Soedinii, 8, 1105-1108 (1993); Ukrainets, I. et al., Chem. Heterocyclic Comp. 33, 600-604, (1997).

The synthesis of various quinoline derivatives is disclosed in WO 97/48694. These compounds are disclosed as capable of binding to nuclear hormone receptors and being useful for stimulating osteoblast proliferation and bone growth. The compounds are also disclosed as being useful in the treatment or prevention of diseases associated with nuclear hormone receptor families.

Various quinoline derivatives in which the benzene ring of the quinoline is substituted with a sulfur group are disclosed in WO 92/18483. These compounds are disclosed as being useful in pharmaceutical formulations and as medicaments.

Quinolone and coumarin derivatives have been disclosed as having use in a variety of applications unrelated to medicine and pharmaceutical formulations. References that describe the preparation of quinolone derivatives for use in photopolymerizable compositions or for luminescent properties include: U.S. Patent No. 5,801,212 issued to Okamoto et al.; JP 8-29973; JP 7-43896; JP 6-9952; JP 63-258903; EP 797376; and DE 23 63 459.

A plethora of substituted quinolinone compounds including quinolinone benzimidazolyl compounds and 4-amino substituted quinolinone benzimidazolyl compounds such as 4-amino-5-fluoro-3-[5-(4-methylpiperazin-1-yl)-1H-benzimidazol-2-yl]quinolin-2(1 H)-one have recently been disclosed in references such as WO 02/22598, WO 2004/043389, WO 2005/047244, U.S. 2004/0220196, U.S. 2005/0137399, WO 2005/046590, and WO 2005/046589. Such compounds are disclosed as inhibiting VEGF-RTKs. Such compounds are also disclosed in published United States patent applications U.S. 2002/0107392 and U.S. 2003/0028018 and U.S. Patent Nos. 6,605,617, 6,774,237, 6,762,194, and 6,800,760. Other such compounds are disclosed along with new uses of such compounds in inhibiting serine/threonine kinases and tyrosine kinases are disclosed in WO 2004/018419, and U.S. 2004/0092535, filed on August 19, 2003, and claiming priority to each of the following provisional applications: U.S. Provisional Application No. 60/405,729 filed on August 23, 2002; U.S. Provisional Application No. 60/426,107 filed on November 13, 2002; U.S. Provisional Application No. 60/426,226 filed on November 13, 2002; U.S. Provisional Application No. 60/426,282 filed on November 13, 2002; U.S. Provisional Application No. 60/428,210 filed on November 21, 2002; U.S. Provisional Application No. 60/460,327 filed on April 3, 2003; U.S. Provisional Application No. filed on April 3, 2003; U.S. Provisional Application No. 60/460,493 filed on April 3, 2003; U.S. Provisional Application No. 60/478,916 filed on June 16, 2003; and U.S. Provisional Application No. 60/484,048 filed on July 1, 2003. Additional disclosure related to quinolinone compounds and uses thereof is set forth in U.S. Provisional Application No. 60/680,722, filed May 13, 2005; U.S. Provisional Application No. 60/681,893, filed May 17, 2005; U.S. Provisional Application No. 60/546,395, filed February 20, 2004; U.S. Provisional Application No. 60/547,103, filed February 23, 2004; U.S. Provisional Application No. 60/554,771, filed March 19, 2004; U.S. Provisional Application No. 60/647,568, filed January 27, 2005; U.S. Provisional Application No. 60/669,245, filed April 6, 2005; U.S. Provisional Application No. 60/538,594, filed January 23, 2004; U.S. Provisional Application No. 60/683,999; filed 5/23/2005; U.S. Patent Application No. 11/061,386, filed February 18, 2005; U.S. Patent Application No. 11/041,191, filed January 21, 2005; and PCT Application No. PCT/US2005/05316, filed February 18, 2005. Heterocyclic compounds related to benzimidazolyl quinolinones have recently been disclosed in WO 02/18383, U.S. 2002/0103230, and U.S. Patent No. 6,756,383. Each of the references in this paragraph is hereby incorporated by reference in its entirety and for all purposes as if fully set forth herein.

Although various quinolinone compounds have been disclosed, new stable formulations, medicaments, and methods for administering such compounds are needed because of the important pharmaceutical applications these compounds have in inhibiting angiogenesis and treating cancer.

### SUMMARY OF THE INVENTION

The present invention provides pharmaceutical formulations of quinolinone compounds such as capsule or tablet formulations that include lactic acid salts of 4-amino-5-fluoro-3-[6-(4-methyl-piperazin-1-yl)-1H-benzimidazol-2-yl]-1 H-quinolin-2-one, and to methods for preparing and using such formulations. The formulations may be produced by dry blending or wet granulation methods.

In one aspect, the present invention provides a pharmaceutical formulation that includes a compound of formula I, a tautomer of the compound, a pharmaceutically acceptable salt of the compound, a pharmaceutically acceptable salt of the tautomer, or a mixture thereof, 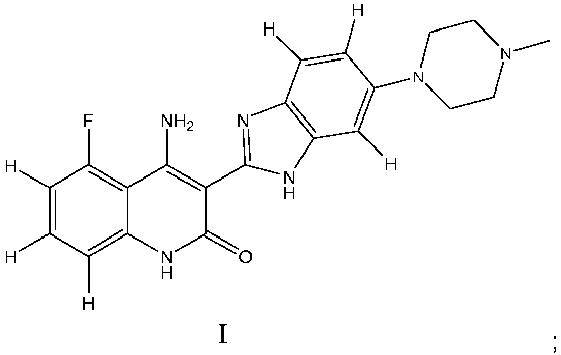 and
at least one ingredient selected from the group consisting of (i) cellulose; (ii) lactose, starch, or a mixture thereof; (iii) povidone; (iv) silicon dioxide or talc; (v) a pharmaceutically acceptable lubricant; and (vi) an ingredient selected from crospovidone, croscarmellose sodium; or sodium starch glycolate.

In another aspect, the present invention provides a pharmaceutical formulation that includes a compound of formula I, a tautomer of the compound, a pharmaceutically acceptable salt of the compound, a pharmaceutically acceptable salt of the tautomer, or a mixture thereof; at least one ingredient selected from the group consisting of cellulose, povidone, silicon dioxide, talc, and a pharmaceutically acceptable lubricant; and at least one ingredient selected from the group consisting of lactose, starch, crospovidone, croscarmellose sodium, and sodium starch glycolate.

In some embodiments, the formulation comprises: (i) cellulose; (ii) silicon dioxide; (iii) stearic acid or a salt of stearic acid; and (iv) at least one ingredient selected from at least one ingredient selected from crospovidone, starch, lactose, croscarmellose sodium, or sodium starch glycolate. In some such embodiments, the formulation comprises crospovidone. In other such embodiments, the formulation comprises a starch such as partially pregelatinized starch. In other embodiments, the formulation comprises lactose.

In some embodiments, the formulation comprises the lactic acid salt of the compound of formula I.

In some embodiments, the formulation is contained within a capsule or tablet. In some such embodiments, the total mass of the compound of formula I, the tautomer of the compound, the lactic acid salt of the compound, the lactic acid salt of the tautomer, or the mixture thereof in the capsule ranges from 25 mg to 500 mg.

In some embodiments, the formulation comprises the lactic acid salt of the compound in an amount ranging from 10% to 50% by weight based on the total weight of the formulation. In some such embodiments, the formulation comprises the lactic acid salt of the compound in an amount ranging from 20% to 45% by weight based on the total weight of the formulation. In some such embodiments, the formulation comprises the lactic acid salt of the compound in an amount ranging from 30% to 40% by weight based on the total weight of the formulation.

In some embodiments, the cellulose used in the formulation is microcrystalline cellulose.

In some embodiments, the formulation comprises the cellulose in an amount ranging from 10% to 70% by weight based on the total weight of the formulation. In some such embodiments, the formulation comprises the cellulose in an amount ranging from 20% to 50% by weight based on the total weight of the formulation, and the formulation comprises crospovidone in an amount ranging from 2% to 6% by weight based on the total weight of the formulation.

In some embodiments, the formulation comprises the starch in an amount ranging from 10% to 40% by weight based on the total weight of the formulation, and the starch is partially pregelatinized starch.

In some embodiments, the formulation comprises the silicon dioxide in an amount ranging from 0.3% to 2% by weight based on the total weight of the formulation.

In some embodiments, the formulation comprises the magnesium stearate in an amount ranging from 0.1 % to 2% by weight based on the total weight of the formulation.

In some embodiments, the formulation comprises the lactic acid salt of the compound in an amount ranging from 30% to 40% by weight based on the total weight of the formulation; the silicon dioxide in an amount ranging from 0.3% to 2% by weight based on the total weight of the formulation, the cellulose in an amount ranging from 25% to 40% of the total weight of the formulation, magnesium stearate in an amount ranging from 0.1 % to 2% by weight based on the total weight of the formulation, and the crospovidone in an amount ranging from 2% to 4% by weight based on the total weight of the formulation.

In some embodiments, the formulation comprises the lactic acid salt of the compound in an amount ranging from 50% to 80% by weight based on the total weight of the formulation; the silicon dioxide in an amount ranging from 0.3% to 2% by weight based on the total weight of the formulation, the cellulose in an amount ranging from 0% to 50% of the total weight of the formulation, magnesium stearate in an amount ranging from 0.1 % to 2% by weight based on the total weight of the formulation, and the starch in an amount ranging from 10% to 40% by weight based on the total weight of the formulation.

The invention also provide pharmaceutical packaging containers. In one embodiment, a packaging container includes a storage vessel comprising two or more capsules or tablets, the capsules or tablets comprising the pharmaceutical formulation of any of the embodiments. In some such embodiments, the storage vessel comprises high density polyethylene. In some such embodiments, the storage vessel includes a cotton or rayon coil, and in some embodiments includes a heat induction seal. In another embodiment, the invention provides a pharmaceutical packaging container that includes a blister package, the blister package comprising at least one capsule or tablet that includes a pharmaceutical formulation of any of the embodiments.

The invention also provides for coating of a tablet of the present invention with a substance selected from the group consisting of sugar, cellulose polymer, and polymethacrylate polymer. In some embodiments this may also include coating the tablet with gelatin or encapsulating the tablet within a gelatin sheath.

The invention also provides for coloring a tablet or capsule of the present invention with a pharmaceutically acceptable coloring agent or opacifier.

In one aspect, the invention provides a method for producing a pharmaceutical formulation. The method includes: (a) blending a first mixture to provide a first blended mixture, the first mixture comprising: (i) a compound of formula I, a tautomer of the compound, a pharmaceutically acceptable salt of the compound, a pharmaceutically acceptable salt of the tautomer, or a mixture thereof, and (ii) at least one ingredient selected from the group consisting of at least one ingredient selected from the group consisting of cellulose; lactose, starch, or a mixture thereof; povidone; silicon dioxide or talc; a pharmaceutically acceptable lubricant; and an ingredient selected from crospovidone, croscarmellose sodium; or sodium starch glycolate. In some such embodiments, the compound of formula I is blended with (i) cellulose; (ii) silicon dioxide; and (iii) an ingredient selected from crospovidone, starch, or lactose. The method may further include (b) blending stearic acid, a salt of stearic acid, or a mixture thereof with the first blended mixture to provide a second blended mixture, and/or (c) forming at least one capsule or at least one tablet from the second blended mixture.

In another aspect, the invention provides a method for producing a pharmaceutical formulation. The method includes: (a) blending a mixture of ingredients to provide a first blended mixture. The first blended mixture includes: i) a compound of formula I, a tautomer of the compound, a pharmaceutically acceptable salt of the compound, a pharmaceutically acceptable salt of the tautomer, or a mixture thereof, (ii) at least one ingredient selected from the group consisting of cellulose; starch; lactose; and povidone; (iii) at least one ingredient selected from the consisting of crospovidone; croscarmellose sodium; and sodium starch glycolate; a granulating fluid selected from the group consisting of aqueous acid; alcohol; aqueous alcohol, or a mixture of any two or more thereof. The method also includes (b) removing the granulating fluid. The method further includes (c) producing a second blended mixture by blending the first blended mixture with at least one additional ingredient selected from the group consisting of: (i) crospovidone, croscarmellose sodium, or sodium starch glycolate; (ii) stearic acid or a salt of stearic acid; and (iii) silicon dioxide or talc. The method may also include (d) forming at least one capsule or at least one tablet from the second blended mixture.

The invention also provides methods for producing pharmaceutical formulation, wherein the pharmaceutical formulation is manufactured using at least one apparatus selected from the group consisting of (i) a fluidized bed granulator equipped with a bottom spray, a top spray, or a tangential spray mechanism; (ii) a high shear granulator; (iii) a low shear granulator; (iv) a roller compactor; and (v) a tablet press.

In some embodiments of the method, the total mass of the compound of formula I, the tautomer of the compound, the pharmaceutically acceptable salt of the compound, the pharmaceutically acceptable salt of the tautomer, or the mixture thereof in the capsule or tablet ranges from 25 mg to 500 mg.

In some embodiments of the method, the second blended mixture comprises a lactic acid salt of the compound of formula I. In other embodiments, the second blended mixture comprises the lactic acid salt of the compound in an amount ranging from 10% to 50% by weight based on the total weight of the second blended mixture.

In some embodiments of the method for producing a pharmaceutical formulation, the cellulose is microcrystalline cellulose. In some embodiments, the starch is pregelatinized starch.

In some methods, the second blended mixture comprises the cellulose in an amount ranging from 10% to 70% by weight based on the total weight of the second blended mixture. In some such embodiments, the second blended mixture comprises the cellulose in an amount ranging from 20% to 50% by weight based on the total weight of the second blended mixture, and the second blended mixture comprises crospovidone in an amount ranging from 2% to 6% by weight based on the total weight of the second blended mixture.

In some methods, the second blended mixture comprises the starch in an amount ranging from 20% to 40% by weight based on the total weight of the second blended mixture, and the starch is partially pregelatinized starch.

In some methods, the second blended mixture comprises the silicon dioxide in an amount ranging from 0.3% to 2% by weight based on the total weight of the second blended mixture.

In some embodiments of the method for producing a pharmaceutical formulation, the second blended mixture comprises the magnesium stearate in an amount ranging from 0.1 % to 2% by weight based on the total weight of the second blended mixture.

In some methods, the second blended mixture comprises the lactic acid salt of the compound in an amount ranging from 50% to 80% by weight based on the total weight of the second blended mixture, in an amount ranging from 55% to 75% by weight based on the total weight of the second blended mixture, or in an amount ranging from 60% to 70% by weight based on the total weight of the second blended mixture.

In some such methods, the silicon dioxide is present in an amount ranging from 0.3% to 2% by weight based on the total weight of the second blended mixture. In other such methods the cellulose is present in an amount ranging from 20% to 45% of the total weight of the second blended mixture. In still other such methods the magnesium stearate is present in an amount ranging from 0.1% to 2% by weight based on the total weight of the second blended mixture. In other such methods the second blended mixture further includes crospovidone in an amount ranging from 2% to 6% by weight based on the total weight of the second blended mixture. In other such embodiments of the methods, the second blended mixture comprises silicon dioxide in an amount ranging from 0.5% to 2% by weight based on the total weight of the second blended mixture, the cellulose in an amount ranging from 20% to 45% of the total weight of the second blended mixture, the magnesium stearate in an amount ranging from 0.5% to 2% by weight based on the total weight of the second blended mixture, and the crospovidone in an amount ranging from 2% to 4% by weight based on the total weight of the second blended mixture.

In some aspects, the invention provides a method for treating cancer and/or inhibiting angiogenesis in a subject. The methods include administering the formulation according to any of the embodiments herein to the subject. In some such embodiments, the formulation comprises a capsule. In other such embodiments, the formulation comprises a tablet.

In some embodiments of the method for treating cancer and/or inhibiting angiogenesis in a subject, the formulation is administered in an amount sufficient to provide a Cₘₐₓ of about 20 to 4000 ng/mL of the compound of formula I, the tautomer of the compound, the lactic acid salt of the compound, the lactic acid salt of the tautomer, or the mixture thereof in the subject's plasma or a Cₘₐₓ of about 40 to 8000 ng/mL of the compound of formula I, the tautomer of the compound, the lactic acid salt of the compound, the lactic acid salt of the tautomer, or the mixture thereof in the subject's blood.

In some embodiments of the method for treating cancer and/or inhibiting angiogenesis in a subject, the formulation is administered in an amount sufficient to provide about 10 to 2,000 ng/mL of the compound of formula I, the tautomer of the compound, the lactic acid salt of the compound, the lactic acid salt of the tautomer, or the mixture thereof in the subject's plasma 24 hours after administration or about 20 to 4,000 ng/mL of the compound of formula I, the tautomer of the compound, the lactic acid salt of the compound, the lactic acid salt of the tautomer, or the mixture thereof in the subject's blood 24 hours after administration.

In some embodiments of the method for treating cancer and/or inhibiting angiogenesis in a subject, the formulation is administered in an amount sufficient to provide to provide an AUC of about 500 to 60,000 ng*h/mL of the compound of formula I, the tautomer of the compound, the lactic acid salt of the compound, the lactic acid salt of the tautomer, or the mixture thereof in the subject's plasma or about 750 to 120,000 ng*h/mL of the compound of formula I, the tautomer of the compound, the lactic acid salt of the compound, the lactic acid salt of the tautomer, or the mixture thereof in the subject's blood.

In some embodiments of the method for treating cancer and/or inhibiting angiogenesis in a subject, the formulation is administered once, twice, three times, or four times daily.

In some embodiments of the method for treating cancer and/or inhibiting angiogenesis in a subject, the amount of the compound of formula I, the tautomer of the compound, the lactic acid salt of the compound, the lactic acid salt of the tautomer, or the mixture thereof administered to the subject ranges from 0.25 to 30 mg/kg body weight of the subject.

In some embodiments of the method for treating cancer and/or inhibiting angiogenesis in a subject, the cancer to be treated is selected from prostate, colorectal, breast, multiple myeloma, pancreatic, small cell carcinoma, acute myelogenous leukemia, chronic myelogenous leukemia, myelo-proliferative disease, nonsmall cell lung, small cell lung, chronic lymphoid leukemia, sarcoma, melanoma, lymphoma, thyroid, neuroendocrine, renal cell, gastric, gastrointestinal stromal, glioma, brain, or bladder cancer. In some embodiments, the cancer has metastasized.

In some embodiments of the method for treating cancer and/or inhibiting angiogenesis in a subject, the method further includes administering the formulation as part of a treatment cycle, wherein the treatment cycle comprises administering the formulation daily for 7, 14, 21, or 28 days, followed by 7 or 14 days without administration of the formulation. In some such embodiments, the treatment cycle comprises administering the amount of the compound daily for 7 days, followed by 7 days without administration of the compound. In some such embodiments, the treatment cycle is repeated one or more times.

Further objects, features and advantages of the invention will be apparent from the drawings and the following detailed description.

### BRIEF DESCRIPTION OF THE DRAWINGS

FIG. 1 is an XRPD pattern characteristic of Form A.
FIG. 2 is a scheme showing various steps used in the manufacture of capsule formulations.

### DETAILED DESCRIPTION OF THE INVENTION

The present invention provides formulationgs of quinolinone compounds. Such formulations may be used to antagonize receptor tyrosine kinases, and, more particularly, to inhibit PDGFRα and PDGFRβ, bFGF and/or VEGF-RTK function. Such formulations may also be used to inhibit other tyrosine kinases and various serine/threonine kinases. The formulations are useful, for example, in treating patients with cancer and/or a need for an inhibitor of VEGF-RTK. The formulations may also be used to treat subject with a need for an inhibitor of angiogenesis.

The following abbreviations and definitions are used throughout this application:

"AUC" is an abbreviation that refers to area under the curve in a graph of the concentration of a compound in blood plasma over time.

"API" is an abbreviation that stands for active pharmaceutical ingredient.

"bFGF" is an abbreviation that stands for basic fibroblast growth factor.

"bFGFR", also referred to as FGFR1, is an abbreviation that stands for a tyrosine kinase that interacts with the fibroblast growth factor FGF.

"Cₘₐₓ" is an abbreviation that refers to the maximum concentration of a compound in the plasma, tissue, or blood of a subject to which the compound has been administered. Cₘₐₓ typically occurs within several hours of administration of a compound to a subject.

"DVS" is an abbreviation that refers to dynamic vapor sorption.

"HDPE" is an abbreviation that refers to high density polyethylene.

"LLOQ" is an abbreviation that refers to lower limit of quantification.

"PDGF" is an abbreviation that stands for platelet derived growth factor. PDGF interacts with tyrosine kinases PDGFRα and PDGFRβ.

"PIB" is an abbreviation that stands for powder-in-bottle formulation.

"RH" is an abbreviation that stands for relative humidity.

"RTK" is an abbreviation that stands for receptor tyrosine kinase.

"VEGF" is an abbreviation that stands for vascular endothelial growth factor.

"VEGF-RTK" is an abbreviation that stands for vascular endothelial growth factor receptor tyrosine kinase.

"XRPD" is an abbreviation that stands for x-ray powder diffraction.

A "pharmaceutically acceptable salt" includes a salt with an inorganic base, organic base, inorganic acid, organic acid, or basic or acidic amino acid. As salts of inorganic bases, the invention includes, for example, alkali metals such as sodium or potassium; alkaline earth metals such as calcium and magnesium or aluminum; and ammonia. As salts of organic bases, the invention includes, for example, trimethylamine, triethylamine, pyridine, picoline, ethanolamine, diethanolamine, and triethanolamine. As salts of inorganic acids, the instant invention includes, for example, hydrochloric acid, hydroboric acid, nitric acid, sulfuric acid, and phosphoric acid. As salts of organic acids, the instant invention includes, for example, formic acid, acetic acid, fumaric acid, oxalic acid, tartaric acid, maleic acid, lactic acid, citric acid, succinic acid, malic acid, methanesulfonic acid, benzenesulfonic acid, and p-toluenesulfonic acid. As salts of basic amino acids, the instant invention includes, for example, arginine, lysine and ornithine. Acidic amino acids include, for example, aspartic acid and glutamic acid.

The term "subject" as used herein refers to any animal that can experience the beneficial effects of the methods of the invention. Thus, a compound of formula I, pharmaceutically acceptable salts thereof, tautomers thereof, or a pharmaceutically acceptable salt of a tautomer can be administered to any animal that can experience the beneficial effects of the compound in accordance with the methods of treating cancer provided by the invention. Preferably, the animal is a mammal, and in particular a human, although the invention is not intended to be so limited. Examples of other suitable animals include, but are not limited to, rats, mice, monkeys, dogs, cats, cattle, horses, pigs, sheep, and the like.

"Treating" within the context of the instant invention means an alleviation of symptoms associated with a disorder or disease, or halt of further progression or worsening of those symptoms, or prevention or prophylaxis of the disease or disorder. For example, within the context of cancer, successful treatment may include an alleviation of symptoms or halting the progression of the disease, as measured by a reduction in the growth rate of a tumor, a halt in the growth of the tumor, a reduction in the size of a tumor, partial or complete remission of the cancer, or increased survival rate or clinical benefit.

In one aspect, the present invention provides a pharmaceutical formulation that comprises a compound of formula I, a tautomer of the compound, a pharmaceutically acceptable salt of the compound, a pharmaceutically acceptable salt of the tautomer, or a mixture thereof, 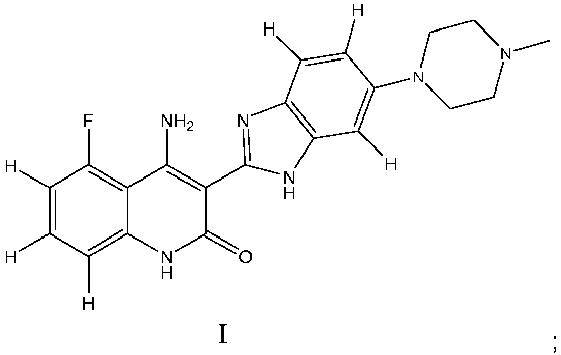 and at least one ingredient selected from the group consisting of (i) cellulose; (ii) lactose, starch, or a mixture thereof; (iii) povidone; (iv) silicon dioxide or talc; (v) a pharmaceutically acceptable lubricant; and (vi) an ingredient selected from crospovidone, croscarmellose sodium; or sodium starch glycolate. In other embodiments, the pharmaceutical formulation includes at least two, three or four ingredients selected from (i) cellulose; (ii) lactose, starch, or a mixture thereof; (iii) povidone; (iv) silicon dioxide or talc; (v) a pharmaceutically acceptable lubricant; and (vi) an ingredient selected from crospovidone, croscarmellose sodium; or sodium starch glycolate.

In another aspect, the present invention provides a pharmaceutical formulation that comprises a compound of formula I, a tautomer of the compound, a pharmaceutically acceptable salt of the compound, a pharmaceutically acceptable salt of the tautomer, or a mixture thereof; and at least one ingredient selected from the group consisting of cellulose, povidone, silicon dioxide, talc, and a pharmaceutically acceptable lubricant; and at least one ingredient selected from the group consisting of lactose, starch, crospovidone, croscarmellose sodium, and sodium starch glycolate.

The formulation may comprise a pharmaceutically acceptable lubricant that reduces the stickiness of powders to metal parts of the capsule filling or tableting machines. Such lubricants are well-known in the art and include a C₁₆₋₂₂ fatty acid, a salt of a C₁₆₋₂₂ fatty acid, a C₁₆₋₂₂ fatty acid ester, a salt of a C₁₆₋₂₂ fatty acid ester; a polyethylene glycol having an average molecular weight of 6,000 to 10,000, and mixtures of any two or more thereof. In some embodiments, the pharmaceutically acceptable lubricant is stearic acid, salts thereof, esters thereof, salts of the esters or mixtures thereof. For example, the formulation may include magnesium stearate, sodium stearate, calcium stearate, zinc stearate, glyceryl monostearate, glyceryl palmitostearate, glyceryl behenate, or sodium stearyl fumarate. As will be understood by those of skill in the art, stearic acid, its salts, esters, and salts of esters include mixtures of C₁₆ and C₁₈ fatty acids, and these mixtures are within the scope of the invention.

The formulation may comprise, consist essentially of, or consist of: the compound of formula I, the tautomer of the compound, the pharmaceutically acceptable salt of the compound, the pharmaceutically acceptable salt of the tautomer, or the mixture thereof and (i) cellulose; (ii) silicon dioxide; (iii) stearic acid, a salt of stearic acid, or a mixture thereof; and (iv) at least ingredient selected from crospovidone, starch, lactose, croscarmellose sodium, or sodium starch glycolate.. In some embodiments, the formulations include (i) microcrystalline cellulose; (ii) silicon dioxide; (iii) magnesium stearate; (iv) at least one ingredient selected from crospovidone, partially pregelatinized starch, and lactose.

The formulation may include the lactic acid salt of the compound of formula I. In some specific embodiments, the lactic acid salt is an anhydrous crystalline form such as Form A which is described and characterized in greater detail in the Examples section of this document.

The formulation may be contained within a capsule or tablet. In some such embodiments, the total mass of the compound of formula I, the tautomer of the compound, the lactic acid salt of the compound, the lactic acid salt of the tautomer, or the mixture thereof in the capsule or tablet ranges from 25 mg to 500 mg. Capsules that may be used include, e.g., white opaque size #0 gelatin capsules such as CS available from Capsugel or HPMC capsules available from Quali-V and Shinogi.

In some embodiments, the formulation comprises the lactic acid salt of the compound in an amount ranging from 10% to 50% by weight based on the total weight of the formulation. In some such embodiments, the formulation comprises the lactic acid salt of the compound in an amount ranging from 20% to 45% by weight based on the total weight of the formulation. In other such embodiments, the formulation comprises the lactic acid salt of the compound in an amount ranging from 30% to 40% by weight based on the total weight of the formulation.

In some embodiments, the cellulose used in the formulation is microcrystalline cellulose. In other embodiments, the cellulose used is silicified microcrystalline cellulose, sodium carboxymethyl cellulose, or hydroxypropyl cellulose.

In some embodiments, the formulation comprises cellulose in an amount ranging from 10% to 70% by weight based on the total weight of the formulation. In some such embodiments, the formulation comprises the cellulose in an amount ranging from 20% to 50% by weight based on the total weight of the formulation, and the formulation comprises crospovidone in an amount ranging from 2% to 6% by weight based on the total weight of the formulation. In some embodiments, the formulation comprises the cellulose in an amount ranging from 20% to 45% by weight based on the total weight of the formulation, and the formulation comprises starch or lactose in an amount ranging from 10% to 40% by weight based on the total weight of the formulation.

In some embodiments, the formulation comprises starch in an amount ranging from 10% to 40% by weight based on the total weight of the formulation, and the starch is partially pregelatinized starch.

The formulations may comprise silicon dioxide in an amount ranging from 0.3% to 2% by weight based on the total weight of the formulation. In some embodiments, the silicon dioxide is present in amounts ranging from 0.2% to 5%, from 0.4% to 4%, from 0.5% to 2%, from 0.75% to 1.5%, or from 0.8% to 1.2% by weight based on the total weight of the formulation. In some embodiments, the silicon dioxide is present in an amount of about 1% by weight based on the total weight of the formulation. In other embodiments the silicon dioxide may be replaced by colloidal silicon dioxide, magnesium silicate, magnesium trisilicate, or talc in the same or similar weight percentages.

The formulations may comprise magnesium stearate in an amount ranging from 0.1 % to 2% by weight based on the total weight of the formulation. In some embodiments, the stearate is present in amounts ranging from 0.2% to 5%, from 0.4% to 4%, from 0.5% to 2%, from 0.75% to 1.5%, or from 0.8% to 1.2% by weight based on the total weight of the formulation. In some embodiments, the stearate is present in an amount of about 1 % by weight based on the total weight of the formulation. In other embodiments the magnesium stearate may be replaced by stearic acid, salts thereof, mixtures thereof, and/or other pharmaceutically acceptable lubricants in the same or similar weight percentages.

In some formulations, the formulation comprises, consists essentially of, or consists of the lactic acid salt of the compound in an amount ranging from 30% to 40% by weight based on the total weight of the formulation; the silicon dioxide in an amount ranging from 0.3% to 2% by weight based on the total weight of the formulation, the cellulose in an amount ranging from 25% to 40% of the total weight of the formulation, the magnesium stearate in an amount ranging from 0.1% to 2% by weight based on the total weight of the formulation, and the crospovidone in an amount ranging from 2% to 4% by weight based on the total weight of the formulation.

In other formulations such as high dose formulations (e.g. 200-500 mg or more API), the composition comprises the lactic acid salt of the compound of formula I in an amount ranging from 50% to 80% by weight based on the total weight of the formulation, from 55% to 75% by weight based on the total weight of the formulation, or from 60% to 70% by weight based on the total weight of the formulation.

In some formulations the composition comprises the lactic acid salt of the compound of formula I in an amount ranging from 50% to 80% by weight based on the total weight of the formulation; the silicon dioxide in an amount ranging from 0.3% to 2% by weight based on the total weight of the formulation, the cellulose in an amount ranging from 0% to 50% of the total weight of the formulation, magnesium stearate in an amount ranging from 0.1% to 2% by weight based on the total weight of the formulation, and the starch in an amount ranging from 10% to 40% by weight based on the total weight of the formulation. In some such embodiments, the formulation comprises the lactic acid salt of the compound in an amount ranging from 55% to 75% by weight based on the total weight of the formulation, the cellulose in an amount ranging from 5% to 40% of the total weight of the formulation, and the starch in an amount ranging from 15% to 30% by weight based on the total weight of the formulation. In other such embodiments, the formulation includes the lactic acid salt of the compound in an amount ranging from 60% to 70% by weight based on the total weight of the formulation and the cellulose in an amount ranging from 5% to 25% of the total weight of the formulation.

In some formulations, the formulation comprises, consists essentially of, or consists of the lactic acid salt of the compound in an amount ranging from 50% to 80% by weight based on the total weight of the formulation; the silicon dioxide in an amount ranging from 0.3% to 2% by weight based on the total weight of the formulation, the cellulose in an amount ranging from 0% to 50% of the total weight of the formulation, magnesium stearate in an amount ranging from 0.1% to 2% by weight based on the total weight of the formulation, and the lactose in an amount ranging from 10% to 40% by weight based on the total weight of the formulation. In some such embodiments, the formulation comprises the lactic acid salt of the compound in an amount ranging from 55% to 75% by weight based on the total weight of the formulation and the cellulose in an amount ranging from 5% to 40% of the total weight of the formulation. In other such embodiments, the formulation comprises the lactic acid salt of the compound in an amount ranging from 60% to 70% by weight based on the total weight of the formulation and the cellulose in an amount ranging from 5% to 40% of the total weight of the formulation.

In some formulations, the formulation further includes an antioxidant, a chelating agent, ascorbic acid, a reducing sugar, or a mixture of any two or more thereof. Suitable anti-oxidants for oral and other formulations include ascorbic acid at, e.g., 0.01 to 0.1 wt %, sodium bisulfite at, e.g., up to 0.65 mg/unit dose, cysteine hydrochloride at, e.g., up to 16 mg/unit dose, methionine, and sodium metabisulfite at, e.g., 0.01 to 0.1 wt %. Other suitable antioxidants for oral and other formulations are reducing sugars containing ketone or aldehyde groups such as fructose, glucose, arabinose and maltose at, e.g., 1 to 55 wt %. Suitable chelating agents include ethylenediaminetetraacetic acid (EDTA) and salts thereof such as calcium disodium ethylenediaminetetraacetic acid (edetate calcium disodium) and tetrasodium ethylenediaminetetraacetic acid (edetate tetrasodium) at, e.g., 0.005 to 0.1 wt %, and sodium citrate at, e.g., 0.3 to 2 wt %.

Pharmaceutical formulations disclosed herein are stable. For example, the amount of degradants of the compound of formula I in formulations of the invention is typically less than 10% by weight based on the total weight of the formulation after storage of the formulation for three months at 40°C and 75% room humidity. In some embodiments, the amount of degradants is less than 8%, less than 5%, less than 4%, less than 3%, less than 2% or even less than 1 % by weight based on the total weight of the formulation after storage of the formulation for three months at 40°C and 75% room humidity.

The invention also provides pharmaceutical packaging containers. In one embodiment, a packaging container includes a storage vessel comprising two or more capsules or tablets, the capsules or tablets comprising the pharmaceutical formulation of any of the embodiments herein. In some such embodiments a plurality of the capsules or tablets comprise the pharmaceutical formulation of any of the embodiments. In some such embodiments, the storage vessel comprises high density polyethylene (HDPE). In some such embodiments, the storage vessel includes a rayon or cotton coil and in some embodiments includes a heat induction seal. In other embodiments the storage vessel comprises high density polyethylene without a rayon coil, but with a heat induction seal. In other embodiments, the invention provides a pharmaceutical packaging container that includes a blister package such as an AI-AI blister package, or a polyvinyl chloride (PVC) package, or a polyvinylidene chloride (PVDC) package, or an Aclar® package. The blister package comprises at least one capsule or tablet that includes a pharmaceutical formulation of any of the embodiments described herein.

In other aspects, the invention may provide for coating a tablet or capsule of the present invention with a coating material such as sugar, cellulose polymer, polymethacrylate polymer. Exemplary cellulose polymer coating agents include but are not limited to methylcellulose, hydroxyethyl cellulose,hydroxyethylmethyl cellulose, hydroxypropyl cellulose, hydroxypropylmethyl cellulose, and ethylcellulose. Suitable polymethacrylate polymer coating agents include but are not limited to methacrylic acid copolymers such as poly(methacrylic acid-methyl methacrylate) and poly(methacrylic acid-ethyl acrylate); ammonio methacrylate copolymer such as poly(ethyl acrylate-methylmethacrylate-trimethylammonioethyl methacrylate chloride); and poly(ethyl acrylate-methyl methacrylate). Other coating materials that may be used include those sold under the tradenames Opadry®, Surelease®, Aquacoat®, and Eudragit®. Another aspect of the invention may include coating a tablet with gelatin or encapsulating a tablet within a gelatin sheath.

In other aspects, the invention provides for the coating material to contain a pharmaceutically acceptable coloring agent, In yet another aspect of the invention, the coating material may contain a pharmaceutically acceptable opacifier. Suitable opacifiers may include titanium dioxide or talc.

In one aspect, the invention provides a method for producing a pharmaceutical formulation. The method includes: (a) blending a first mixture to provide a first blended mixture, the first mixture comprising: (i) a compound of formula I, a tautomer of the compound, a pharmaceutically acceptable salt of the compound, a pharmaceutically acceptable salt of the tautomer, or a mixture thereof, and (ii) at least one ingredient selected from the group consisting of cellulose; lactose, starch, or a mixture thereof; povidone; silicon dioxide or talc; a pharmaceutically acceptable lubricant; and an ingredient selected from crospovidone, croscarmellose sodium; or sodium starch glycolate. In some such embodiments, the compound of formula I is blended with (i) cellulose; (ii) silicon dioxide; and (iii) an ingredient selected from crospovidone, starch, or lactose. The method may further include (b) blending stearic acid, a salt of stearic acid, or a mixture thereof with the first blended mixture to provide a second blended mixture, and/or (c) forming at least one capsule or at least one tablet from the second blended mixture.

In another aspect, the invention provides a method for producing a pharmaceutical formulation. The method includes: (a) blending a mixture of ingredients to provide a first blended mixture. The first blended mixture includes: i) a compound of formula I, a tautomer of the compound, a pharmaceutically acceptable salt of the compound, a pharmaceutically acceptable salt of the tautomer, or a mixture thereof, (ii) at least one ingredient selected from the group consisting of cellulose; starch; lactose; and povidone; (iii) at least one ingredient selected from the consisting of crospovidone; croscarmellose sodium; and sodium starch glycolate; a granulating fluid selected from the group consisting of aqueous acid; alcohol; aqueous alcohol, or a mixture of any two or more thereof. For example, the granulation fluid of the method may be water or aqueous hydrochloric acid. The method also includes (b) removing the granulating fluid, e.g., by drying. The method also includes (c) producing a second blended mixture by blending the first blended mixture with at least one additional ingredient selected from the group consisting of: (i) crospovidone, croscarmellose sodium, or sodium starch glycolate; (ii) stearic acid or a salt of stearic acid; and (iii) silicon dioxide or talc. Steps (a), (b) and (c) may be performed sequentially or simultaneously, or step (c) may be performed prior to step (b). The method may also include (d) forming at least one capsule or at least one tablet from the second blended mixture.

Methods of producing the pharmaceutical formations disclosed herein may include the use of various equipment well known to those of skill in the art. Suitable equipment includes a fluidized bed granulator equipped with a bottom spray, a top spray, or a tangential spray mechanism; a high shear granulator; a low shear granulator; a roller compactor; a sizer; a capsule filler, and/or a tablet press. Thus, for example, fluid bed granulators that may be used are those available from Niro Pharma Systems such as the Sirocco®, Multi-processor®, MP-Micro®, STREA-1®, MP-1 Multi-processor®, as well as fluid bed granulator/dryer/coater available from Glatt; high-shear granulators available from Niro Pharma Systems such as the Collette Gral®, UltimaGral®, PMA Pharma Matrix®, from Bohle such as the Bohle mini granulator, and from Glatt Air Techniques such as the Glatt-Powrex Vertical Granulator; low-shear granulators such as the V-Blender and Hobart mixer/granulator; and roller compactors from Fitzpatrick Chilsonators, the Gerteis Micro-, Mini-, and Macro-pactors, and the Vector TFC Roller Compactor; sizing equipment is available as the Quadro from Comil, the Hammer mill from Fitzpatrick Chilsonators, and an oscillator available from several vendors; capsule fillers from MG2 (MG), Bosch (GKF), and IMA (Zanasi); and/or a tablet press such as that from Manesty, Fette, and Courtoy.

In some embodiments of the method, the total mass of the compound of formula I, the tautomer of the compound, the pharmaceutically acceptable salt of the compound, the pharmaceutically acceptable salt of the tautomer, or the mixture thereof in the capsule or tablet ranges from 25 mg to 500 mg.

In some embodiments, the second blended mixture comprises a lactic acid salt of the compound of formula I. In other embodiments, the second blended mixture comprises the lactic acid salt of the compound in an amount ranging from 10% to 50% by weight based on the total weight of the second blended mixture, in an amount ranging from 20% to 45% by weight based on the total weight of the second blended mixture, or in an amount ranging from 30% to 40% by weight based on the total weight of the second blended mixture.

In some embodiments of the method for producing a pharmaceutical formulation, the cellulose is microcrystalline cellulose. In some embodiments, the starch is pregelatinized starch.

In some methods, the second blended mixture comprises the cellulose in an amount ranging from 10% to 70% by weight based on the total weight of the second blended mixture. In some such embodiments, the second blended mixture comprises the cellulose in an amount ranging from 20% to 50% by weight based on the total weight of the second blended mixture, and the second blended mixture comprises crospovidone in an amount ranging from 2% to 6% by weight based on the total weight of the second blended mixture. In some embodiments, the second blended mixture comprises the cellulose in an amount ranging from 20% to 50% by weight based on the total weight of the second blended mixture, and the second blended mixture comprises starch or lactose in an amount ranging from 10% to 40% by weight based on the total weight of the second blended mixture.

In some methods, the second blended mixture comprises the starch in an amount ranging from 20% to 40% by weight based on the total weight of the second blended mixture, and the starch is partially pregelatinized starch.

In some methods, the second blended mixture comprises the silicon dioxide in an amount ranging from 0.3% to 2% by weight based on the total weight of the second blended mixture. In other embodiments, the silicon dioxide is present in amounts ranging from 0.2% to 5%, from 0.4% to 4%, from 0.5% to 2%, from 0.75% to 1.25%, or from 0.8% to 1.2% by weight based on the total weight of the second blended mixture. In some embodiments, the silicon dioxide is present in an amount of about 1 % by weight based on the total weight of the second blended mixture.

In some methods, the second blended mixture comprises a salt of stearic acid such as magnesium stearate. For example, in some methods magnesium stearate is present in an amount ranging from 0.1 % to 2% by weight based on the total weight of the second blended mixture. In other embodiments, the stearate is present in amounts ranging from 0.2% to 5%, from 0.4% to 4%, from 0.5% to 1.5%, from 0.75% to 1.25%, or from 0.8% to 1.2% by weight based on the total weight of the second blended mixture. In some embodiments, the stearate is present in an amount of about 1 % or 1 % by weight based on the total weight of the second blended mixture.

In some methods, the second blended mixture comprises the lactic acid salt of the compound in an amount ranging from 50% to 80% by weight based on the total weight of the second blended mixture, in an amount ranging from 55% to 75% by weight based on the total weight of the second blended mixture, or in an amount ranging from 60% to 70% by weight based on the total weight of the second blended mixture.

In some such methods, the silicon dioxide is present in an amount ranging from 0.3% to 2% by weight based on the total weight of the second blended mixture. In other such methods the cellulose is present in an amount ranging from 20% to 45% of the total weight of the second blended mixture. In still other such methods the magnesium stearate is present in an amount ranging from 0.1 % to 2% by weight based on the total weight of the second blended mixture. In other such methods the second blended mixture further includes crospovidone in an amount ranging from 2% to 6% by weight based on the total weight of the second blended mixture. In other such embodiments of the methods, the second blended mixture comprises silicon dioxide in an amount ranging from 0.5% to 2% by weight based on the total weight of the second blended mixture, the cellulose in an amount ranging from 20% to 45% of the total weight of the second blended mixture, the magnesium stearate in an amount ranging from 0.5% to 2% by weight based on the total weight of the second blended mixture, and the crospovidone in an amount ranging from 2% to 4% by weight based on the total weight of the second blended mixture.

The invention also provides a method for treating cancer and/or inhibiting angiogenesis in a subject. The methods include administering the formulation according to any of the embodiments to the subject. In some such embodiments, the formulation comprises a capsule or tablet. Suitable subjects include mammals such as rats, mice, monkeys and other primates, dogs, cats, cattle, horses, pigs, sheep, and the like. In some embodiments, the subject is a human, and in some such embodiments is a human cancer patient. In some embodiments, the formulation is delivered orally as a capsule or tablet to a patient such as a human cancer patient.

The formulation may be administered in an amount sufficient to provide a Cₘₐₓ of about 20 to 4000 ng/mL of the compound of formula I, the tautomer of the compound, the lactic acid salt of the compound, the lactic acid salt of the tautomer, or the mixture thereof in the subject's plasma or a Cₘₐₓ of about 40 to 8000 ng/mL of the compound of formula I, the tautomer of the compound, the lactic acid salt of the compound, the lactic acid salt of the tautomer, or the mixture thereof in the subject's blood. In some embodiments, the amount administered is sufficient to provide a Cₘₐₓ of about 35 to 2000 ng/mL in the subject's plasma or a Cₘₐₓ of about 70 to 4000 ng/mL in the subject's blood, a Cₘₐₓ of about 50 to 500 ng/mL in the subject's plasma or a Cₘₐₓ of about 100 to 1000 ng/mL in the subject's blood, a Cₘₐₓ of about 50 to 250 ng/mL in the subject's plasma or a Cₘₐₓ of about 100 to 500 ng/mL in the subject's blood, a Cₘₐₓ of about 75 to 150 ng/mL in the subject's plasma or a Cₘₐₓ of about 150 to 300 ng/mL in the subject's blood, a Cₘₐₓ of about 100 to 2000 ng/mL in the subject's plasma or a Cₘₐₓ of about 200 to 4000 ng/mL in the subject's blood, or a Cₘₐₓ of 100 to 1000 ng/mL in the subject's plasma or a Cₘₐₓ of about 200 to 2000 ng/mL in the subject's blood.

The formulation may also administered in an amount sufficient to provide about 10 to 2,000 ng/mL of the compound of formula I, the tautomer of the compound, the lactic acid salt of the compound, the lactic acid salt of the tautomer, or the mixture thereof in the subject's plasma 24 hours after administration or about 20 to 4,000 ng/mL of the compound of formula I, the tautomer of the compound, the lactic acid salt of the compound, the lactic acid salt of the tautomer, or the mixture thereof in the subject's blood 24 hours after administration. In some embodiments, the amount administered is sufficient to provide about 20 to 1,000 ng/mL in the subject's plasma 24 hours after administration or about 40 to 2,000 ng/mL in the subject's blood 24 hours after administration, about 40 to 500 ng/mL in the subject's plasma 24 hours after administration or about 80 to 1,000 ng/mL in the subject's blood 24 hours after administration, or about 40 to 250 ng/mL in the subject's plasma 24 hours after administration or about 80 to 500 ng/mL in the subject's blood 24 hours after administration.

The formulation may yet also be administered in an amount sufficient to provide to provide an AUC of about 500 to 60,000 ng*h/mL of the compound of formula I, the tautomer of the compound, the lactic acid salt of the compound, the lactic acid salt of the tautomer, or the mixture thereof in the subject's plasma or about 750 to 120,000 ng*h/mL of the compound of formula I, the tautomer of the compound, the lactic acid salt of the compound, the lactic acid salt of the tautomer, or the mixture thereof in the subject's blood. In other such embodiments, the amount administered is sufficient to provide an AUC of about 1,000 to 30,000 ng*h/mL in the subject's plasma or about 1,500 to 60,000 ng*h/mL in the subject's blood. In other such embodiments, the AUC is about 2,000 to 15,000 ng*h/mL in the subject's plasma or about 3,000 to 30,000 ng*h/mL in the subject's blood.

The formulations of the invention may be in a capsule or tablet sufficient to provide at least one of
(a) a Cₘₐₓ of about 20 to 4000 ng/mL of the compound of formula I, the tautomer of the compound, the lactic acid salt of the compound, the lactic acid salt of the tautomer, or the mixture thereof in a subject's plasma or a Cₘₐₓ of about 40 to 8000 ng/mL of the compound of formula I, the tautomer of the compound, the lactic acid salt of the compound, the lactic acid salt of the tautomer, or the mixture thereof in the subject's blood after administration to the subject,
(b) about 10 to 2,000 ng/mL of the compound of formula I, the tautomer of the compound, the lactic acid salt of the compound, the lactic acid salt of the tautomer, or the mixture thereof in a subject's plasma 24 hours after administration or about 20 to 4,000 ng/mL of the compound of formula I, the tautomer of the compound, the lactic acid salt of the compound, the lactic acid salt of the tautomer, or the mixture thereof in the subject's blood 24 hours after administration to the subject, or
(c) an AUC of about 500 to 60,000 ng*h/mL of the compound of formula I, the tautomer of the compound, the lactic acid salt of the compound, the lactic acid salt of the tautomer, or the mixture thereof in a subject's plasma or about 750 to 120,000 ng*h/mL of the compound of formula I, the tautomer of the compound, the lactic acid salt of the compound, the lactic acid salt of the tautomer, or the mixture thereof in the subject's blood after administration to the subject.

The formulations may also be in a capsule or tablet sufficient to provide at least one of
(a) a Cₘₐₓ of about 50 to 500 ng/mL of the compound of formula I, the tautomer of the compound, the lactic acid salt of the compound, the lactic acid salt of the tautomer, or the mixture thereof in the subject's plasma or a Cₘₐₓ of about 100 to 1000 ng/mL of the compound of formula I, the tautomer of the compound, the lactic acid salt of the compound, the lactic acid salt of the tautomer, or the mixture thereof in the subject's blood after administration,
(b) about 20 to 1,000 ng/mL of the compound of formula I, the tautomer of the compound, the lactic acid salt of the compound, the lactic acid salt of the tautomer, or the mixture thereof in the subject's plasma 24 hours after administration or about 40 to 2,000 ng/mL of the compound of formula I, the tautomer of the compound, the lactic acid salt of the compound, the lactic acid salt of the tautomer, or the mixture thereof in the subject's blood 24 hours after administration, or
(c) an AUC of about 1,000 to 30,000 ng*h/mL of the compound of formula I, the tautomer of the compound, the lactic acid salt of the compound, the lactic acid salt of the tautomer, or the mixture thereof in the subject's plasma or about 1,500 to 60,000 ng*h/mL of the compound of formula I, the tautomer of the compound, the lactic acid salt of the compound, the lactic acid salt of the tautomer, or the mixture thereof in the subject's blood after administration.

The formulations may still further be in a capsule or tablet sufficient to provide at least one of
(a) a Cₘₐₓ of about 50 to 250 ng/mL of the compound of formula I, the tautomer of the compound, the lactic acid salt of the compound, the lactic acid salt of the tautomer, or the mixture thereof in the subject's plasma or a Cₘₐₓ of about 100 to 500 ng/mL of the compound of formula I, the tautomer of the compound, the lactic acid salt of the compound, the lactic acid salt of the tautomer, or the mixture thereof in the subject's blood after administration,
(b) about 40 to 500 ng/mL of the compound of formula I, the tautomer of the compound, the lactic acid salt of the compound, the lactic acid salt of the tautomer, or the mixture thereof in the subject's plasma 24 hours after administration or about 80 to 1,000 ng/mL of the compound of formula I, the tautomer of the compound, the lactic acid salt of the compound, the lactic acid salt of the tautomer, or the mixture thereof in the subject's blood 24 hours after administration, or
(c) an AUC of about 2,000 to 15,000 ng*h/mL of the compound of formula I, the tautomer of the compound, the lactic acid salt of the compound, the lactic acid salt of the tautomer, or the mixture thereof in the subject's plasma or about 3,000 to 30,000 ng*h/mL of the compound of formula I, the tautomer of the compound, the lactic acid salt of the compound, the lactic acid salt of the tautomer, or the mixture thereof in the subject's blood after administration.

The formulations may still also be in a capsule or tablet sufficient to provide at least one of
(a) a Cₘₐₓ of about 75 to 150 ng/mL of the compound of formula I, the tautomer of the compound, the lactic acid salt of the compound, the lactic acid salt of the tautomer, or the mixture thereof in the subject's plasma or a Cₘₐₓ of about 150 to 300 ng/mL of the compound of formula I, the tautomer of the compound, the lactic acid salt of the compound, the lactic acid salt of the tautomer, or the mixture thereof in the subject's blood after administration, or
(b) about 40 to 250 ng/mL of the compound of formula I, the tautomer of the compound, the lactic acid salt of the compound, the lactic acid salt of the tautomer, or the mixture thereof in the subject's plasma 24 hours after administration or about 80 to 500 ng/mL of the compound of formula I, the tautomer of the compound, the lactic acid salt of the compound, the lactic acid salt of the tautomer, or the mixture thereof in the subject's blood 24 hours after administration.

In some embodiments, each unit dose of the formulation is sufficient to provide a Cₘₐₓ of about 100 to 2000 ng/mL of the compound of formula I, the tautomer of the compound, the lactic acid salt of the compound, the lactic acid salt of the tautomer, or the mixture thereof in the subject's plasma or a Cₘₐₓ of about 200 to 4000 ng/mL of the compound of formula I, the tautomer of the compound, the lactic acid salt of the compound, the lactic acid salt of the tautomer, or the mixture thereof in the subject's blood; or a Cₘₐₓ of 100 to 1000 ng/mL of the compound of formula I, the tautomer of the compound, the lactic acid salt of the compound, the lactic acid salt of the tautomer, or the mixture thereof in the subject's plasma or a Cₘₐₓ of about 200 to 2000 ng/mL of the compound in the subject's blood after administration.

In some embodiments of the method for treating cancer and/or inhibiting angiogenesis in a subject, the formulation is administered once, twice, three times, or four times daily.

The amount of the compound of formula I, the tautomer of the compound, the lactic acid salt of the compound, the lactic acid salt of the tautomer, or the mixture thereof administered to the subject may range from 0.25 to 30 mg/kg body weight of the subject. In other embodiments, the amount administered to the subject may range from about 25 to 1500 mg/subject per day, from about 100 to 1000 mg/subject per day, or from about 200 to 500 mg/subject per day

In some embodiments of the method for treating cancer and/or inhibiting angiogenesis in a subject, the cancer to be treated is selected from prostate, colorectal, breast, multiple myeloma, pancreatic, small cell carcinoma, acute myelogenous leukemia, chronic myelogenous leukemia, myelo-proliferative disease, nonsmall cell lung, small cell lung, chronic lymphoid leukemia, sarcoma, melanoma, lymphoma, thyroid, neuroendocrine, renal cell, gastric, gastrointestinal stromal, glioma, brain, refractory multiple myeloma, or bladder cancer. In some embodiments, the cancer has metastasized.

In some embodiments of the method for treating cancer and/or inhibiting angiogenesis in a subject, the method further includes administering the formulation as part of a treatment cycle, wherein the treatment cycle comprises administering the formulation daily for 7, 14, 21, or 28 days, followed by 7 or 14 days without administration of the formulation. In some such embodiments, the treatment cycle comprises administering the amount of the compound daily for 7 days, followed by 7 days without administration of the compound. In some such embodiments, the treatment cycle is repeated one or more times.

Ingredients in addition those described herein may be included in the formulations of the present invention. Such additional or alternative ingredients are described, for example, in "Remington's Pharmaceutical Sciences" Mack Pub. Co., New Jersey (1991), which is incorporated herein by reference. Such additional or alternative ingredients include, but are not limited to: methylcellulose, hydroxyethyl cellulose, hydroxyethylmethyl cellulose, hydroxypropyl cellulose, hydroxypropylmethyl cellulose, ethylcellulose, sodium lauryl sulfate, cab-o-sil, Avicel PH, poly(ethyl acrylate-methyl methacrylate), methacrylic acid copolymers such as but not limited to poly(methacrylic acid-methyl methacrylate) and poly(methacrylic acid-ethyl methacrylate), and aminomethacrylate copolymers such as but not limited to poly(ethyl acrylate-methylmethacrylate-trimethylammonioethyl methacrylate chloride).

The formulations of the invention may be designed for to be short-acting, fast-releasing, long-acting, and sustained-releasing. Thus, the pharmaceutical formulations may also be formulated for controlled release or for slow release.

A therapeutically effective dose refers to that amount of the compound that results in amelioration of symptoms. Specific dosages may be adjusted depending on conditions of disease, the age, body weight, general health conditions, sex, diet of the subject, dose intervals, administration routes, excretion rate, and combinations of drugs. Any of the above dosage forms containing effective amounts are well within the bounds of routine experimentation and therefore, well within the scope of the instant invention. A therapeutically effective dose may vary depending upon the route of administration and dosage form. The preferred compound or compounds of the instant invention is a formulation that exhibits a high therapeutic index. The therapeutic index is the dose ratio between toxic and therapeutic effects which can be expressed as the ratio between LD₅₀ and ED₅₀. The LD₅₀ is the dose lethal to 50% of the population and the ED₅₀ is the dose therapeutically effective in 50% of the population. The LD₅₀ and ED₅₀ are determined by standard pharmaceutical procedures in animal cell cultures or experimental animals.

An RTK disorder, or RTK-mediated disease, which may be treated by those methods provided, include any biological disorder or disease in which an RTK is implicated, or which inhibition of and RTK potentiates a biochemical pathway that is defective in the disorder or disease state. Examples of such diseases are cancers such as prostate, colorectal, breast, multiple myeloma, pancreatic, small cell carcinoma, acute myelogenous leukemia, chronic myelogenous leukemia, or myelo-proliferative disease.

Scheme 1 depicts one exemplary synthetic route for the synthesis of a compound used in the formulations of the present invention and should not be interpreted to limit the invention in any manner.

In any formulation, method, or packaging of the present invention it is contemplated where capsules are so provided, tablets may also be provided and where tablets are so provided, capsules may also be provided.

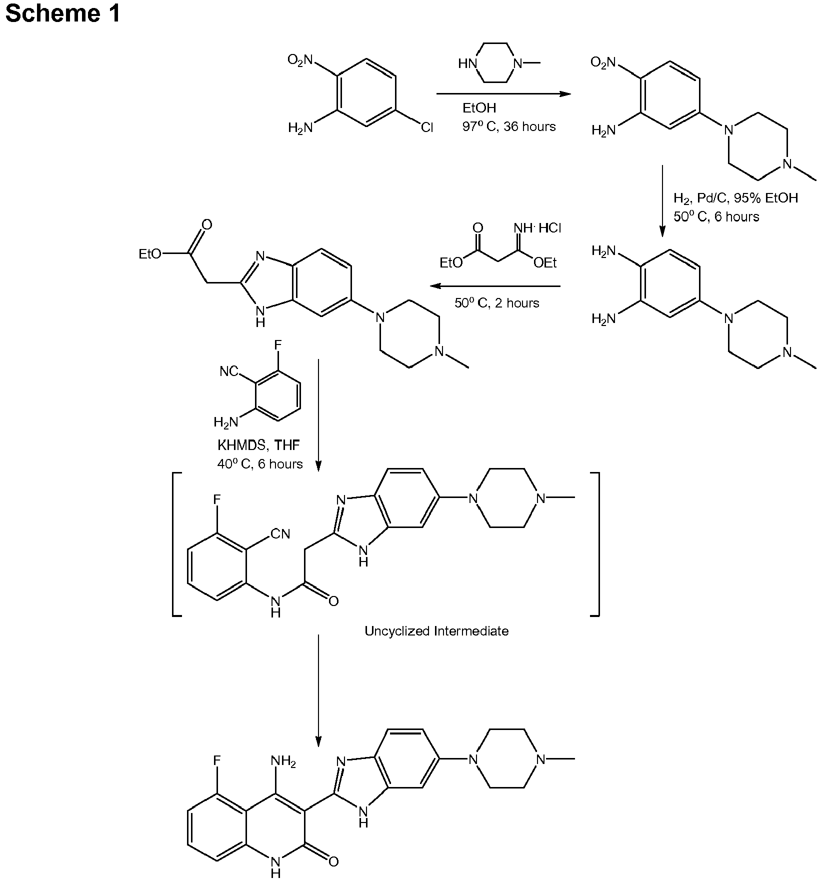

It should be understood that the organic compounds according to the invention may exhibit the phenomenon of tautomerism. As the chemical structures within this specification can only represent one of the possible tautomeric forms at a time, it should be understood that the invention encompasses any tautomeric form of the drawn structure. For example, the compound having the formula I is shown below with one tautomer, Tautomer Ia:

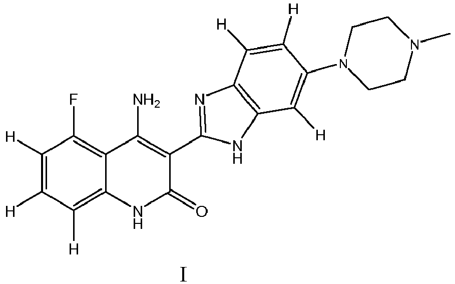

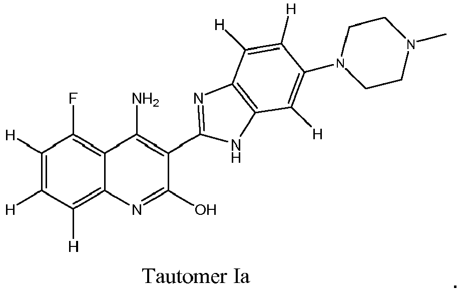

Other tautomers of the compound having the formula I, Tautomer Ib and Tautomer Ic, are shown below:

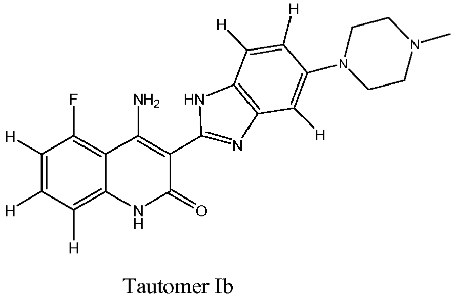

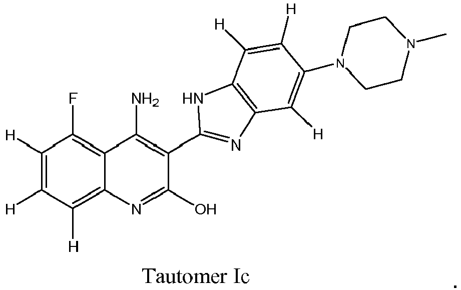

The present invention, thus generally described, will be understood more readily by reference to the following examples, which are provided by way of illustration and are not intended to be limiting of the present invention.

### EXAMPLES

The following abbreviations are used in the Examples:
- EtOH:: Ethanol
- H₂O:: Water
- HCl:: Hydrochloric acid
- HPLC:: High Performance Liquid Chromatography
- KHMDS:: Potassium bis(trimethylsilyl)amide
- LiHMDS:: Lithium bis(trimethylsilyl)amide
- NaHMDS:: Sodium bis(trimethylsilyl)amide
- NaOH:: Sodium hydroxide
- N₂:: Nitrogen
- TBME:: t-Butyl methyl ether
- THF:: Tetrahydrofuran

Nomenclature for the Example compounds was provided using ACD Name version 5.07 software (November 14, 2001) available from Advanced Chemistry Development, Inc., ChemInnovation NamExpert + Nomenclator™ brand software available from ChemInnovation Software, Inc., and AutoNom version 2.2 available in the ChemOffice® Ultra software package version 7.0 available from CambridgeSoft Corporation (Cambridge, MA). Some of the compounds and starting materials were named using standard IUPAC nomenclature.

Various starting materials may be obtained from commercial sources and prepared by methods known to one of skill in the art.

### Example 1

### Synthesis of 5-(4-Methyl-piperazin-1-yl)-2-nitroaniline

### Procedure A

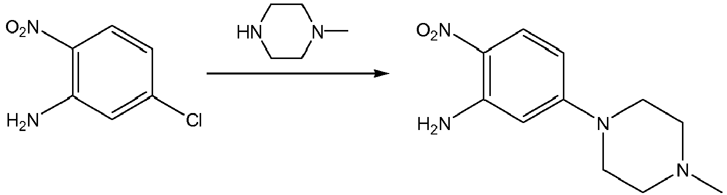

5-Chloro-2-nitroaniline (500 g, 2.898 mol) and 1-methyl piperazine (871 g, 8.693 mol) were placed in a 2000 mL flask fitted with a condenser and purged with N₂. The flask was placed in an oil bath at 100°C and heated until the 5-chloro-2-nitroaniline was completely reacted (typically overnight) as determined by HPLC. After HPLC confirmed the disappearance of the 5-chloro-2-nitroaniline, the reaction mixture was poured directly (still warm) into 2500 mL of room temperature water with mechanical stirring. The resulting mixture was stirred until it reached room temperature and then it was filtered. The yellow solid thus obtained was added to 1000 mL of water and stirred for 30 minutes. The resulting mixture was filtered, and the resulting solid was washed with TBME (500 mL, 2X) and then was dried under vacuum for one hour using a rubber dam. The resulting solid was transferred to a drying tray and dried in a vacuum oven at 50°C to a constant weight to yield 670 g (97.8%) of the title compound as a yellow powder.

### Procedure B

5-Chloro-2-nitroaniline (308.2 g, 1.79 mol) was added to a 4-neck 5000 mL round bottom flask fitted with an overhead stirrer, condenser, gas inlet, addition funnel, and thermometer probe. The flask was then purged with N₂. 1-Methylpiperazine (758.1 g, 840 mL, 7.57 mol) and 200 proof ethanol (508 mL) were added to the reaction flask with stirring. The flask was again purged with N₂, and the reaction was maintained under N₂. The flask was heated in a heating mantle to an internal temperature of 97°C (+/- 5°C) and maintained at that temperature until the reaction was complete (typically about 40 hours) as determined by HPLC. After the reaction was complete, heating was discontinued and the reaction was cooled to an internal temperature of about 20°C to 25°C with stirring, and the reaction was stirred for 2 to 3 hours. Seed crystals (0.20 g, 0.85 mmol) of 5-(4-methyl-piperazin-1-yl)-2-nitroaniline were added to the reaction mixture unless precipitation had already occurred. Water (2,450 mL) was added to the stirred reaction mixture over a period of about one hour while the internal temperature was maintained at a temperature ranging from about 20°C to 30°C. After the addition of water was complete, the resulting mixture was stirred for about one hour at a temperature of 20°C to 30°C. The resulting mixture was then filtered, and the flask and filter cake were washed with water (3 x 2.56 L). The golden yellow solid product was dried to a constant weight of 416 g (98.6% yield) under vacuum at about 50°C in a vacuum oven.

### Procedure C

5-Chloro-2-nitroaniline (401 g, 2.32 mol) was added to a 4-neck 12 L round bottom flask fitted with an overhead stirrer, condenser, gas inlet, addition funnel, and thermometer probe. The flask was then purged with N₂. 1-Methylpiperazine (977 g, 1.08 L, 9.75 mol) and 100% ethanol (650 mL) were added to the reaction flask with stirring. The flask was again purged with N₂, and the reaction was maintained under N₂. The flask was heated in a heating mantle to an internal temperature of 97°C (+/- 5°C) and maintained at that temperature until the reaction was complete (typically about 40 hours) as determined by HPLC. After the reaction was complete, heating was discontinued and the reaction was cooled to an internal temperature of about 80°C with stirring, and water (3.15 L) was added to the mixture via an addition funnel over the period of 1 hour while the internal temperature was maintained at 82°C (+/- 3°C). After water addition was complete, heating was discontinued and the reaction mixture was allowed to cool over a period of no less than 4 hours to an internal temperature of 20-25°C. The reaction mixture was then stirred for an additional hour at an internal temperature of 20-30°C. The resulting mixture was then filtered, and the flask and filter cake were washed with water (1 x 1 L), 50% ethanol (1 x 1 L), and 95% ethanol (1 x 1 L). The golden yellow solid product was placed in a drying pan and dried to a constant weight of 546 g (99% yield) under vacuum at about 50°C in a vacuum oven.

### Example 2

### Synthesis of [6-(4-Methyl-piperazin-1-yl)-1H-benzimidazol-2-yl]-acetic acid ethyl ester

### Procedure A

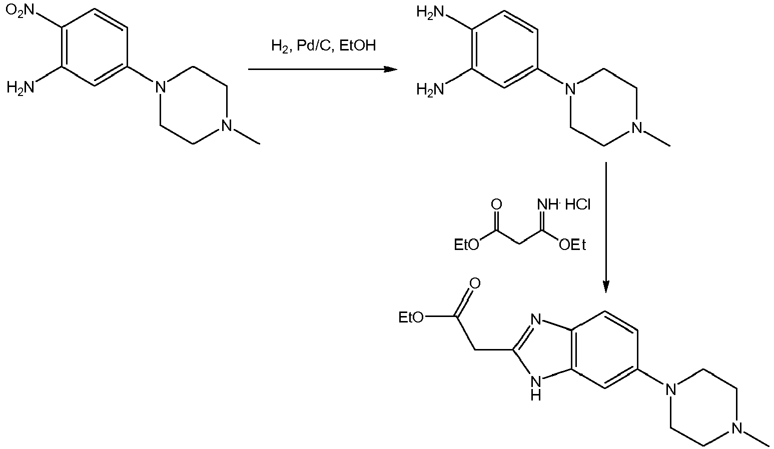

A 5000 mL, 4-neck flask was fitted with a stirrer, thermometer, condenser, and gas inlet/outlet. The equipped flask was charged with 265.7 g (1.12 mol. 1.0 eq) of 5-(4-methyl-piperazin-1-yl)-2-nitroaniline and 2125 mL of 200 proof EtOH. The resulting solution was purged with N₂ for 15 minutes. Next, 20.0 g of 5% Pd/C (50% H₂O w/w) was added. The reaction was vigorously stirred at 40-50°C (internal temperature) while H₂ was bubbled through the mixture. The reaction was monitored hourly for the disappearance of 5-(4-methyl-piperazin-1-yl)-2-nitroaniline by HPLC. The typical reaction time was 6 hours.

After all the 5-(4-methyl-piperazin-1-yl)-2-nitroaniline had disappeared from the reaction, the solution was purged with N₂ for 15 minutes. Next, 440.0 g (2.25 mol) of ethyl 3-ethoxy-3-iminopropanoate hydrochloride was added as a solid. The reaction was stirred at 40-50°C (internal temperature) until the reaction was complete. The reaction was monitored by following the disappearance of the diamino compound by HPLC. The typical reaction time was 1-2 hours. After the reaction was complete, it was cooled to room temperature and filtered through a pad of Celite filtering material. The Celite filtering material was washed with absolute EtOH (2 x 250 mL), and the filtrate was concentrated under reduced pressure providing a thick brown/orange oil. The resulting oil was taken up in 850 mL of a 0.37% HCl solution. Solid NaOH (25 g) was then added in one portion, and a precipitate formed. The resulting mixture was stirred for 1 hour and then filtered. The solid was washed with H₂O (2 x 400 mL) and dried at 50°C in a vacuum oven providing 251.7 g (74.1 %) of [6-(4-methyl-piperazin-1-yl)-1 H-benzoimidazol-2-yl]-acetic acid ethyl ester as a pale yellow powder.

### Procedure B

A 5000 mL, 4-neck jacketed flask was fitted with a mechanical stirrer, condenser, temperature probe, gas inlet, and oil bubbler. The equipped flask was charged with 300 g (1.27 mol) of 5-(4-methyl-piperazin-1-yl)-2-nitroaniline and 2400 mL of 200 proof EtOH (the reaction may be and has been conducted with 95% ethanol and it is not necessary to use 200 proof ethanol for this reaction). The resulting solution was stirred and purged with N₂ for 15 minutes. Next, 22.7 g of 5% Pd/C (50% H₂O w/w) was added to the reaction flask. The reaction vessel was purged with N₂ for 15 minutes. After purging with N₂, the reaction vessel was purged with H₂ by maintaining a slow, but constant flow of H₂ through the flask. The reaction was stirred at 45-55°C (internal temperature) while H₂ was bubbled through the mixture until the 5-(4-methyl-piperazin-1-yl)-2-nitroaniline was completely consumed as determined by HPLC. The typical reaction time was 6 hours.

After all the 5-(4-methyl-piperazin-1-yl)-2-nitroaniline had disappeared from the reaction, the solution was purged with N₂ for 15 minutes. The diamine intermediate is air sensitive so care was taken to avoid exposure to air. 500 g (2.56 mol) of ethyl 3-ethoxy-3-iminopropanoate hydrochloride was added to the reaction mixture over a period of about 30 minutes. The reaction was stirred at 45-55°C (internal temperature) under N₂ until the diamine was completely consumed as determined by HPLC. The typical reaction time was about 2 hours. After the reaction was complete, the reaction was filtered while warm through a pad of Celite. The reaction flask and Celite were then washed with 200 proof EtOH (3 x 285 mL). The filtrates were combined in a 5000 mL flask, and about 3300 mL of ethanol was removed under vacuum producing an orange oil. Water (530 mL) and then 1 M HCL (350 mL) were added to the resulting oil, and the resulting mixture was stirred. The resulting solution was vigorously stirred while 30% NaOH (200 mL) was added over a period of about 20 minutes maintaining the internal temperature at about 25-30°C while the pH was brought to between 9 and 10. The resulting suspension was stirred for about 4 hours while maintaining the internal temperature at about 20-25°C. The resulting mixture was filtered, and the filter cake was washed with H₂O (3 x 300 mL). The collected solid was dried to a constant weight at 50°C under vacuum in a vacuum oven providing 345.9 g (90.1 %) of [6-(4-methyl-piperazin-1-yl)-1H-benzoimidazol-2-yl]-acetic acid ethyl ester as a pale yellow powder. In an alternative work up procedure, the filtrates were combined and the ethanol was removed under vacuum until at least about 90% had been removed. Water at a neutral pH was then added to the resulting oil, and the solution was cooled to about 0°C. An aqueous 20% NaOH solution was then added slowly with rapid stirring to bring the pH up to 9.2 (read with pH meter). The resulting mixture was then filtered and dried as described above. The alternative work up procedure provided the light tan to light yellow product in yields as high as 97%.

### Example 3

### Method for Reducing Water Content of [6-(4-Methyl-piperazin-1-yl)-1H-benzoimidazol-2-yl]-acetic acid ethyl ester

[6-(4-Methyl-piperazin-1-yl)-1H-benzimidazol-2-yl]-acetic acid ethyl ester (120.7 grams) that had been previously worked up and dried to a water content of about 8-9% H₂O was placed in a 2000 mL round bottom flask and dissolved in absolute ethanol (500 mL). The amber solution was concentrated to a thick oil using a rotary evaporator with heating until all solvent was removed. The procedure was repeated two more times. The thick oil thus obtained was left in the flask and placed in a vacuum oven heated at 50°C overnight. Karl Fisher analysis results indicated a water content of 5.25%. The lowered water content obtained by this method provided increased yields in the procedure of Example 4. Other solvents such as toluene and THF may be used in place of the ethanol for this drying process.

### Example 4

### Synthesis of 4-Amino-5-fluoro-3-[6-(4-methyl-piperazin-1-yl)-1H-benzimidazol-2-yl]-1H-quinolin-2-one

### Procedure A

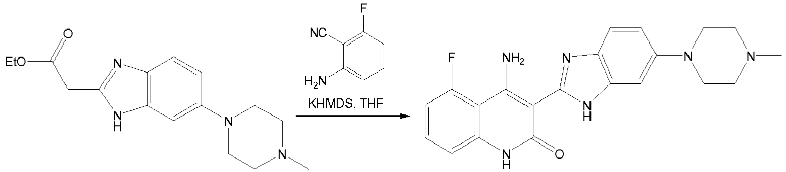

[6-(4-Methyl-piperazin-1-yl)-1H-benzimidazol-2-yl]-acetic acid ethyl ester (250 g, 820 mmol) (dried with ethanol as described above) was dissolved in THF (3800 mL) in a 5000 mL flask fitted with a condenser, mechanical stirrer, temperature probe, and purged with argon. 2-Amino-6-fluoro-benzonitrile (95.3 g, 700 mmol) was added to the solution, and the internal temperature was raised to 40°C. When all the solids had dissolved and the solution temperature had reached 40°C, solid KHMDS (376.2 g, 1890 mmol) was added over a period of 5 minutes. When addition of the potassium base was complete, a heterogeneous yellow solution was obtained, and the internal temperature had risen to 62°C. After a period of 60 minutes, the internal temperature decreased back to 40°C, and the reaction was determined to be complete by HPLC (no starting material or uncyclized intermediate was present). The thick reaction mixture was then quenched by pouring it into H₂O (6000 mL) and stirring the resulting mixture until it had reached room temperature. The mixture was then filtered, and the filter pad was washed with water (1000 mL 2X). The bright yellow solid was placed in a drying tray and dried in a vacuum oven at 50°C overnight providing 155.3 g (47.9%) of the desired 4-amino-5-fluoro-3-[6-(4-methyl-piperazin-1-yl)-1 H-benzimidazol-2-yl]-1H-quinolin-2-one.

### Procedure B

A 5000 mL 4-neck jacketed flask was equipped with a distillation apparatus, a temperature probe, a N₂ gas inlet, an addition funnel, and a mechanical stirrer. [6-(4-Methyl-piperazin-1-yl)-1H-benzimidazol-2-yl]-acetic acid ethyl ester (173.0 g, 570 mmol) was charged into the reactor, and the reactor was purged with N₂ for 15 minutes. Dry THF (2600 mL) was then charged into the flask with stirring. After all the solid had dissolved, solvent was removed by distillation (vacuum or atmospheric (the higher temperature helps to remove the water) using heat as necessary. After 1000 mL of solvent had been removed, distillation was stopped and the reaction was purged with N₂. 1000 mL of dry THF was then added to the reaction vessel, and when all solid was dissolved, distillation (vacuum or atmospheric) was again conducted until another 1000 mL of solvent had been removed. This process of adding dry THF and solvent removal was repeated at least 4 times (on the 4^{th} distillation, 60% of the solvent is removed instead of just 40% as in the first 3 distillations) after which a 1 mL sample was removed for Karl Fischer analysis to determine water content. If the analysis showed that the sample contained less than 0.20% water, then reaction was continued as described in the next paragraph. However, if the analysis showed more than 0.20% water, then the drying process described above was continued until a water content of less than 0.20% was achieved.

After a water content of less than or about 0.20% was achieved using the procedure described in the previous paragraph, the distillation apparatus was replaced with a reflux condenser, and the reaction was charged with 2-amino-6-fluoro-benzonitrile (66.2 g, 470 mmol)(in some procedures 0.95 equivalents is used). The reaction was then heated to an internal temperature of 38-42°C. When the internal temperature had reached 38-42°C, KHMDS solution (1313 g, 1.32 mol, 20% KHMDS in THF) was added to the reaction via the addition funnel over a period of 5 minutes maintaining the internal temperature at about 38-50°C during the addition. When addition of the potassium base was complete, the reaction was stirred for 3.5 to 4.5 hours (in some examples it was stirred for 30 to 60 minutes and the reaction may be complete within that time) while maintaining the internal temperature at from 38-42°C. A sample of the reaction was then removed and analyzed by HPLC. If the reaction was not complete, additional KHMDS solution was added to the flask over a period of 5 minutes and the reaction was stirred at 38-42°C for 45-60 minutes (the amount of KHMDS solution added was determined by the following: If the IPC ratio is < 3.50, then 125 mL was added; if 10.0 ≥ IPC ratio ≥ 3.50, then 56 mL was added; if 20.0 ≥ IPC ratio ≥ 10, then 30 mL was added. The IPC ratio is equal to the area corresponding to 4-amino-5-fluoro-3-[6-(4-methyl-piperazin-1-yl)-1 H-benzimidazol-2-yl]-1 H-quinolin-2-one) divided by the area corresponding to the uncyclized intermediate). Once the reaction was complete (IPC ratio > 20), the reactor was cooled to an internal temperature of 25-30°C, and water (350 mL) was charged into the reactor over a period of 15 minutes while maintaining the internal temperature at 25-35°C (in one alternative, the reaction is conducted at 40°C and water is added within 5 minutes. The quicker quench reduces the amount of impurity that forms over time). The reflux condenser was then replaced with a distillation apparatus and solvent was removed by distillation (vacuum or atmospheric) using heat as required. After 1500 mL of solvent had been removed, distillation was discontinued and the reaction was purged with N₂. Water (1660 mL) was then added to the reaction flask while maintaining the internal temperature at 20-30°C. The reaction mixture was then stirred at 20-30°C for 30 minutes before cooling it to an internal temperature of 5-10°C and then stirring for 1 hour. The resulting suspension was filtered, and the flask and filter cake were washed with water (3 x 650 mL). The solid thus obtained was dried to a constant weight under vacuum at 50°C in a vacuum oven to provide 103.9 g (42.6% yield) of 4-amino-5-fluoro-3-[6-(4-methyl-piperazin-1-yl)-1H-benzimidazol-2-yl]-1H-quinolin-2-one as a yellow powder.

### Procedure C

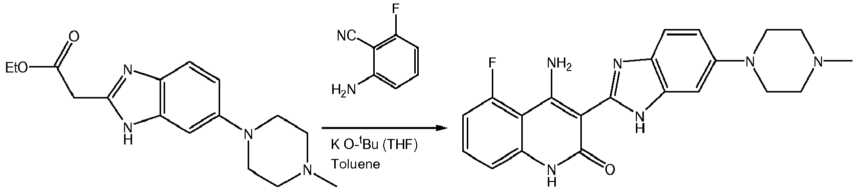

[6-(4-Methyl-piperazin-1-yl)-1H-benzimidazol-2-yl]-acetic acid ethyl ester (608 g, 2.01 mol) (dried) and 2-amino-6-fluoro-benzonitrile (274 g, 2.01 mol) were charged into a 4-neck 12 L flask seated on a heating mantle and fitted with a condenser, mechanical stirrer, gas inlet, and temperature probe. The reaction vessel was purged with N₂, and toluene (7.7 L) was charged into the reaction mixture while it was stirred. The reaction vessel was again purged with N₂ and maintained under N₂. The internal temperature of the mixture was raised until a temperature of 63°C (+/- 3°C) was achieved. The internal temperature of the mixture was maintained at 63°C (+/- 3°C) while approximately 2.6 L of toluene was distilled from the flask under reduced pressure (380 +/- 10 torr, distilling head t = 40°C (+/- 10°C) (Karl Fischer analysis was used to check the water content in the mixture. If the water content was greater than 0.03%, then another 2.6 L of toluene was added and distillation was repeated. This process was repeated until a water content of less than 0.03% was achieved). After a water content of less than 0.03% was reached, heating was discontinued, and the reaction was cooled under N₂ to an internal temperature of 17-19°C. Potassium t-butoxide in THF (20% in THF; 3.39 kg, 6.04 moles potassium t-butoxide) was then added to the reaction under N₂ at a rate such that the internal temperature of the reaction was kept below 20°C. After addition of the potassium t-butoxide was complete, the reaction was stirred at an internal temperature of less than 20°C for 30 minutes. The temperature was then raised to 25°C, and the reaction was stirred for at least 1 hour. The temperature was then raised to 30°C, and the reaction was stirred for at least 30 minutes. The reaction was then monitored for completion using HPLC to check for consumption of the starting materials (typically in 2-3 hours, both starting materials were consumed (less than 0.5% by area % HPLC)). If the reaction was not complete after 2 hours, another 0.05 equivalents of potassium t-butoxide was added at a time, and the process was completed until HPLC showed that the reaction was complete. After the reaction was complete, 650 mL of water was added to the stirred reaction mixture. The reaction was then warmed to an internal temperature of 50°C and the THF was distilled away (about 3 L by volume) under reduced pressure from the reaction mixture. Water (2.6 L) was then added dropwise to the reaction mixture using an addition funnel. The mixture was then cooled to room temperature and stirred for at least 1 hour. The mixture was then filtered, and the filter cake was washed with water (1.2 L), with 70% ethanol (1.2 L), and with 95% ethanol (1.2 L). The bright yellow solid was placed in a drying tray and dried in a vacuum oven at 50°C until a constant weight was obtained providing 674 g (85.4%) of the desired 4-amino-5-fluoro-3-[6-(4-methyl-piperazin-1-yl)-1H-benzimidazol-2-yl]-1H-quinolin-2-one.

### Example 5

### Purification of 4-Amino-5-fluoro-3-[6-(4-methyl-piperazin-1-yl)-1H-benzimidazol-2-yl]-1H-quinolin-2-one

A 3000 mL 4-neck flask equipped with a condenser, temperature probe, N₂ gas inlet, and mechanical stirrer was placed in a heating mantle. The flask was then charged with 4-amino-5-fluoro-3-[6-(4-methyl-piperazin-1-yl)-1 H-benzimidazol-2-yl]-1 H-quinolin-2-one (101.0 g, 0.26 mol), and the yellow solid was suspended in 95% ethanol (1000 mL) and stirred. In some cases an 8:1 solvent ratio is used. The suspension was then heated to a gentle reflux (temperature of about 76°C) with stirring over a period of about 1 hour. The reaction was then stirred for 45-75 minutes while refluxed. At this point, the heat was removed from the flask and the suspension was allowed to cool to a temperature of 25-30°C. The suspension was then filtered, and the filter pad was washed with water (2 x 500 mL). The yellow solid was then placed in a drying tray and dried in a vacuum oven at 50°C until a constant weight was obtained (typically 16 hours) to obtain 97.2 g (96.2%) of the purified product as a yellow powder.

### Example 6

### Preparation of Lactic Acid salt of 4-Amino-5-fluoro-3-[6-(4-methyl-piperazin-1-yl)-1H-benzimidazol-2-yl]-1H-quinolin-2-one

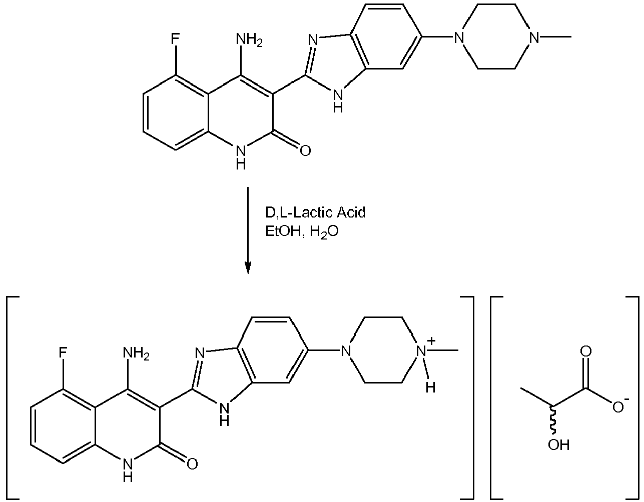

A 3000 mL 4-necked jacketed flask was fitted with a condenser, a temperature probe, a N₂ gas inlet, and a mechanical stirrer. The reaction vessel was purged with N₂ for at least 15 minutes and then charged with 4-amino-5-fluoro-3-[6-(4-methyl-piperazin-1-yl)-1 H-benzimidazol-2-yl]-1 H-quinolin-2-one (484 g, 1.23 mol). A solution of D,L-Lactic acid (243.3 g, 1.72 mol of monomer-see the following paragraph), water (339 mL), and ethanol (1211 mL) was prepared and then charged to the reaction flask. Stirring was initiated at a medium rate, and the reaction was heated to an internal temperature of 68-72°C. The internal temperature of the reaction was maintained at 68-72°C for 15-45 minutes and then heating was discontinued. The resulting mixture was filtered through a 10-20 micron frit collecting the filtrate in a 12 L flask. The 12 L flask was equipped with an internal temperature probe, a reflux condenser, an addition funnel, a gas inlet an outlet, and an overhead stirrer. The filtrate was then stirred at a medium rate and heated to reflux (internal temperature of about 78°C). While maintaining a gentle reflux, ethanol (3,596 mL) was charged to the flask over a period of about 20 minutes. The reaction flask was then cooled to an internal temperature ranging from about 64-70°C within 15-25 minutes and this temperature was maintained for a period of about 30 minutes. The reactor was inspected for crystals. If no crystals were present, then crystals of the lactic acid salt of 4-amino-5-fluoro-3-[6-(4-methyl-piperazin-1-yl)-1 H-benzimidazol-2-yl]-1 H-quinolin-2-one (484 mg, 0.1 mole %) were added to the flask, and the reaction was stirred at 64-70°C for 30 minutes before again inspecting the flask for crystals. Once crystals were present, stirring was reduced to a low rate and the reaction was stirred at 64-70°C for an additional 90 minutes. The reaction was then cooled to about 0°C over a period of about 2 hours, and the resulting mixture was filtered through a 25-50 micron fritted filter. The reactor was washed with ethanol (484 mL) and stirred until the internal temperature was about 0°C. The cold ethanol was used to wash the filter cake, and this procedure was repeated 2 more times. The collected solid was dried to a constant weight at 50°C under vacuum in a vacuum oven yielding 510.7 g (85.7%) of the crystalline yellow lactic acid salt of 4-amino-5-fluoro-3-[6-(4-methyl-piperazin-1-yl)-1H-benzimidazol-2-yl]-1H-quinolin-2-one. This procedure provided Form A of the lactic acid salt of the compound. A rubber dam or inert conditions were typically used during the filtration process. While the dry solid did not appear to be very hygroscopic, the wet filter cake tends to pick up water and become sticky. Precautions were taken to avoid prolonged exposure of the wet filter cake to the atmosphere.

Commercial lactic acid generally contains about 8-12% w/w water, and contains dimers and trimers in addition to the monomeric lactic acid. The mole ratio of lactic acid dimer to monomer is generally about 1.0:4.7. Commercial grade lactic acid may be used in the process described in the preceding paragraph as the monolactate salt preferentially precipitates from the reaction mixture.

### Example 7

### X-Ray Analysis of Lactic Acid Salt, Form A

### Preliminary Crystallinity Studies

Preliminary XRPD (X-ray powder diffraction) analyses were carried out on a Shimadzu XRD-6000 X-ray powder diffractometer using Cu Kα radiation. The instrument is equipped with a fine focus X-ray tube. The tube voltage and amperage were set to 40 kV and 40 mA, respectively. The divergence and scattering slits were set at 1 ° and the receiving slit was set at 0.15 mm. Diffracted radiation was detected by a NaI scintillation detector. A theta-two theta continuous scan at 3°/minute (0.4 seconds/0.02° step) from 2.5 to 40°C was used. 4-Amino-5-fluoro-3-[6-(4-methylpiperazin-1-yl)-1H-benzimidazol-2-yl]-1H-quinolin-2-one lactic acid salt was found to exhibit a high degree of crystallinity and have a distinct powder X-ray diffraction.

### Further XRPD Characterization of 4-Amino-5-fluoro-3-[6-(4-methylpiperazin-1-yl)-1H-benzimidazol-2-yl]-1H-quinolin-2-one Lactic Acid, Form A

XRPD was carried out with a Philips X'Pert powder diffractometer (Copper Kα radiation). Metallic sample holders of 0.4 or 0.8 mm depth were used (TTK type). Due to the high potency of the investigated drug substance, the sample holders were covered with a thin Kapton foil after preparation in a laminar flow bench. The wavelength of the CuKα1 radiation is 1.54060 A. The X-ray tube was operated at a voltage of 40 kV, and a current of 40 mA. A step size of 0.02°, and a counting time of 2.0 to 2.4 s per step were applied. Due to packing density of the powder in the sample holder, the recorded intensity may be variable, and a small amorphous background resulting from the Kapton foil is difficult to distinguish from any amorphous drug substance that might be present in a sample obtained from a crystallization experiment.

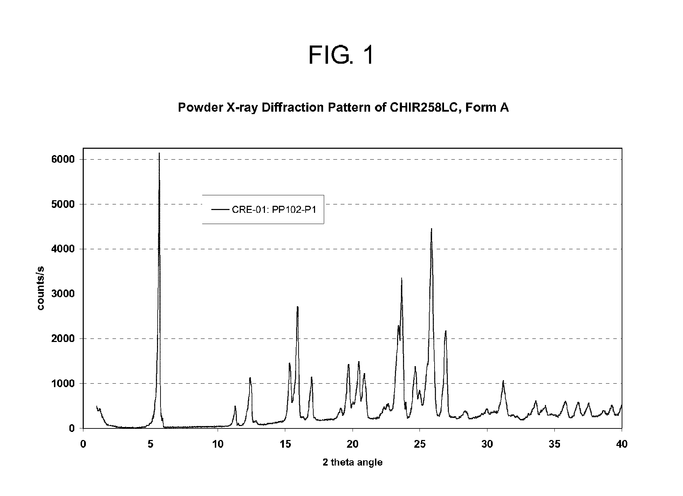

The XRPD pattern of Form A is provided in FIG. 1. Relatively prominent two-theta peaks were observed at about 5.7, about 11.3, about 12.4, about 15.3, about 15.9, about 17.0, about 19.1, about 19.7, about 20.5, about 20.9, about 22.8, about 23.4, about 23.7, about 24.7, about 25.0, about 25.9, about 26.9, and about 31.2 degrees.

### Example 8

### Hygroscopicity of Form A

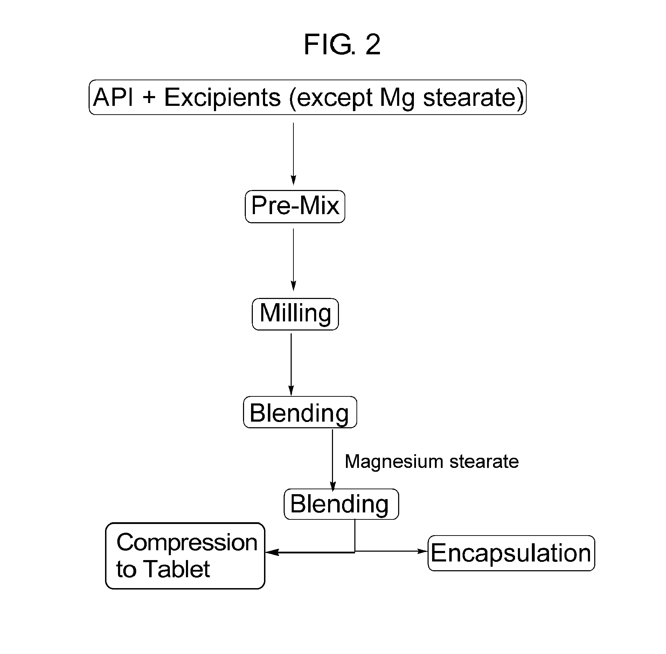

Investigation of 4-amino-5-fluoro-3-[6-(4-methylpiperazin-1-yl)-1H-benzimidazol-2-yl]-1 H-quinolin-2-one lactic acid, Form A, in a DVS experiment shows that below about 80% RH the investigated Form A is not hygroscopic (see Table 1). All DVS measurements were carried out at 2.5% relative humidity change per hour. However, exposure to RH conditions above 90% led to a significant water uptake, which was not completely reversible during the applied measurement time. Furthermore, the water uptake was not complete when at 4500 minutes the relative humidity was scanned back from 95% to 50%. The results of the DVS measurement are shown in FIGS. 2 and 3.

**Table 1. Moisture Induced Weight Change in Salts of 4-Amino-5-fluoro-3-[6-(4-methylpiperazin-1-yl)-1H-benzimidazol-2-yl]-1H-quinolin-2-one.**

| **Salt Form** | **Moisture induced % weight change** | | |
|---|---|---|---|
| | 55% RH | 85% RH | 95% RH |
| Lactate trial 1 | 0.61 | 1.39 | 12.84 |
| Lactate trial 2 | 0.13 | 0.42 | 2.76 |
| Lactate trial 3 | 0.08 | 0.15 | 0.24 |
| Mesylate trial 1 | 1.88 | 2.38 | 4.12 |
| Mesylate trial 2 | 6.32 | 7.65 | 22.63 |
| Malate trial 1 | 0.64 | 1.49 | 2.71 |
| Malate trial 2 | 0.16 | 0.34 | 0.56 |
| Malate trial 3 | 0.08 | 0.18 | 0.30 |

### Example 9

### Formulations of 4-Amino-5-fluoro-3-[6-(4-methyl-piperazin-1-yl)-1H-benzimidazol-2-yl]-1H-quinolin-2-one

Capsule formulations were prepared using the general method shown in FIG. 2. The lactic acid salt of 4-amino-5-fluoro-3-[6-(4-methyl-piperazin-1-yl)-1 H-benzimidazol-2-yl]-1 H-quinolin-2-one was prepared as described above and the anhydrous crystalline form was preferably used to prepare the formulations described herein (Form A). Excipients used in the formulations include lactose monohydrate (e.g., FAST FLO #316, from Foremost Whey Products and DMV Corp.), microcrystalline cellulose (e.g., AVICEL 101, from FMC Corp.), Partially pregelatinized starch (e.g., STARCH 1500, from Colorcon, Inc.), povidone (from ISP or Base), crospovidone (e.g., POLYPLASDONE XL, from ISP), silicon dioxide (e.g., SYLOID 244FP, from Grace Davison, or Cabot), and magnesium stearate (e.g., from Mallinckrodt).

Twelve capsule formulations (Compositions 1-12) were prepared having the weight percent amounts of ingredients shown in Table 2. The capsules were adjusted to have 15 mg of API (compound of formula I) each. Three additional formulations were prepared on a scale of about 1.5 kg at two different strengths (25 mg and 100 mg (Composition 13), and 30 mg and 100 mg (Compositions 14 and 15)). The ingredients and quantities used to prepare the compositions are shown in Tables 3-5.

Briefly, each of the ingredients except the magnesium stearate were combined and premixed prior to milling. After milling and blending, magnesium stearate was added and the mixture was blended a second time. After blending with the added magnesium stearate, the compositions were encapsulated to provide the 25 mg, 30 mg, and 100 mg compositions. For the 25 and 30 mg composition levels, size 2 Swedish orange opaque capsules were used, and for the 100 mg compositions size 0 grey opaque gelatin (CS, Capsugel) or HPMC (QUALI-V, Shanogi) capsules were used. The same or a similar procedure may be used to prepare capsules other than the sizes shown in Tables 2-5. For example, the same procedure may be used to prepare capsules of 25 mg, 30 mg, 50 mg, 100 mg, 150 mg, 200 mg, 250 mg, 300 mg, 350 mg, 400 mg, 450 mg, and 500 mg by simply adjusting the appropriate capsule size and the amount of the ingredients in the composition as will be apparent to those of skill in the art.

The stability of each formulation shown in Table 2 was evaluated over 3 months while storing the formulations at 40°C/75% room humidity. Impurities and degradants were found to be less than 0.6% for all formulations over this time and were less than 0.4% for most formulations.

**Table 2. Dry Blend Formulations**

| **Comp. ID No.** | | **(% w/w)** | | | | | | |
|---|---|---|---|---|---|---|---|---|
| | **Capsule Type** | **Lactose** | **CP** | **SLS** | **PS** | **SiO₂** | **MCC** | **API** |
| 1 | G | 0 | 4 | 1 | 30 | 0 | 30 | 35 |
| 2 | G | 0 | 0 | 1 | 30 | 1 | 0 | 68 |
| 3 | H | 30 | 0 | 1 | 30 | 0 | 30 | 9 |
| 4 | G | 30 | 0 | 1 | 0 | 0 | 0 | 69 |
| 5 | G | 0 | 4 | 0 | 0 | 0 | 30 | 66 |
| 6 | G | 30 | 4 | 0 | 30 | 1 | 0 | 35 |
| 7 | H | 30 | 4 | 1 | 0 | 1 | 30 | 34 |
| 8 | H | 0 | 4 | 1 | 0 | 1 | 0 | 94 |
| 9 | H | 0 | 0 | 0 | 0 | 0 | 0 | 100 |
| 10 | H | 0 | 0 | 0 | 30 | 1 | 30 | 39 |
| 11 | G | 30 | 0 | 0 | 0 | 1 | 30 | 39 |
| 12 | H | 30 | 4 | 0 | 30 | 0 | 0 | 36 |

| | | | | | | | | |
|---|---|---|---|---|---|---|---|---|
| G = Hard Gelatin capsule; H = Hydroxymethylpropyl cellulose capsule PS = Partially Pregelatinized Starch CP = Crospovidone SLS = Sodium lauryl sulfate MCC = Microcrystalline cellulose API = Compound of formula I | | | | | | | | |

Based on these results, three additional formulations (compositions 13-15) were prepared as described above. Each composition was found to have desirable stability and dissolution properties as shown in Tables 6-15. Therefore, various embodiments include any of the compositions described herein. No degradation products were detected in any of the formulations. Each of compositions 13-15 had excellent dissolution characteristics. For example, 80 % of composition 1 was dissolved in 10 minutes, 85% of composition 2 was dissolved in 10 minutes, and 85% of composition 3 was dissolved in 20 minutes. These are all well within standards imposed by the Food and Drug Administration in which 85% must be dissolved within 45 minutes. In some embodiments, composition 14 is the formulation of choice. In other embodiments, composition 13 or composition 15 is the formulation of choice.

In-process testing was conducted to determine the uniformity of the blends, the particle size distribution (PSD) of the blend, and the bulk/tap density for all compositions. Three packaging configurations were used to store the capsules after preparation. In one configuration, capsules were stored in a high density polyethylene (HDPE) bottle with a rayon coil and a heat-induction seal. In a second configuration, capsules were stored in a HDPE bottle without a rayon coil, but with a heat-induction seal. In a third configuration, the capsules were stored in an AI-AI blister package. Stability testing was performed with respect to uniformity of the contents, the appearance, and dissolution properties. HPLC assays were also used to study stability of the capsule formulations.

**Table 3. Capsule Composition 13.**

| **Ingredient** | | **25 mg** | **100 mg** |
|---|---|---|---|
| | **% (w/w)** | **mg/capsule** | **mg/capsule** |
| Lactic acid salt of 4-amino-5-fluoro-3-[6-(4-methyl-piperazin-1-yl)-1H-benzimidazol-2-yl]-1H-quinolin-2-one | 34.0 | 30.8 | 123.0 |
| Crospovidone | 4.0 | 3.6 | 14.5 |
| Silicon dioxide | 1.0 | 0.9 | 3.6 |
| Microcrystalline cellulose | 60.0 | 54.3 | 217.1 |
| Magnesium stearate | 1.0 | 0.9 | 3.6 |
| Total | 100.0 | 90.4 | 361.8 |

**Table 4. Capsule Composition 14.**

| **Ingredient** | **% (w/w)** |
|---|---|
| Lactic acid salt of 4-amino-5-fluoro-3-[6-(4-methyl-piperazin-1-yl)-1H-benzimidazol-2-yl]-1H-quinolin-2-one | 38.0 |
| Partially Pregelatinized starch (Starch 1500) | 30.0 |
| Silicon dioxide | 1.0 |
| Microcrystalline cellulose | 30.0 |
| Magnesium stearate | 1.0 |
| Total | 100.0 |

**Table 5. Capsule Composition 15.**

| **Ingredient** | **% (w/w)** |
|---|---|
| Lactic acid salt of 4-amino-5-fluoro-3-[6-(4-methyl-piperazin-1-yl)-1H-benzimidazol-2-yl]-1H-quinolin-2-one | 34.0 |
| Lactose | 30.0 |
| Silicon dioxide | 1.0 |
| Microcrystalline cellulose | 30.0 |
| Magnesium stearate | 1.0 |
| Total | 100.0 |

**Table 6. Stability Data for Capsule Compositions Stored for Three Months at 40°C (75% RH).***

| **Formulation** | **Weeks** | **Water By KF (%)** | **% assay Cmpd 1** | **% Area** | |
|---|---|---|---|---|---|
| | | | | **RRT 0.56** | **RRT 1.27** |
| Composition 13 (25 mg) | Initial | 4.3 | 98.9 | - | 0.16 |
| | 4 | 4.8 | 99.2 | - | 0.16 |
| | 8 | 4.0 | 103.5 | - | 0.16 |
| | 12 | 4.5 | 100.3 | - | 0.16 |
| Composition 13 (100 mg) | Initial | 4.2 | 98.1 | 0.05 | 0.16 |
| | 4 | 4.1 | 99.3 | - | 0.16 |
| | 8 | 3.6 | 96.4 | - | 0.17 |
| | 12 | 3.9 | 99.0 | - | 0.16 |
| Composition 14 (30 mg) | Initial | 4.4 | 99.2 | - | 0.14 |
| | 4 | 4.5 | 102.9 | - | 0.14 |
| | 8 | 3.8 | 100.4 | - | 0.14 |
| | 12 | 4.1 | 99.9 | - | 0.14 |
| Composition 14 (100 mg) | Initial | 4.2 | 104.6 | - | 0.14 |
| | 4 | 4.2 | 103.1 | - | 0.14 |
| | 8 | 3.9 | 102.2 | - | 0.14 |
| | 12 | 4.9 | 102.6 | - | 0.14 |
| Composition 15 (30 mg) | Initial | 3.6 | 100.7 | - | 0.15 |
| | 4 | 3.4 | 102.1 | - | 0.14 |
| | 8 | 3.7 | 101.2 | - | 0.15 |
| | 12 | 3.3 | 99.3 | - | 0.15 |
| Composition 15 (100 mg) | Initial | 3.6 | 99.8 | 0.05 | 0.14 |
| | 4 | 3.4 | 100.5 | - | 0.15 |
| | 8 | 3.3 | 99.6 | - | 0.15 |
| | 12 | 3.3 | 99.3 | - | 0.15 |

| | | | | | |
|---|---|---|---|---|---|
| *The packaging configuration was a HDPE bottle without rayon coil and heat-induction seal. | | | | | |

**Table 7. Stability Data for Capsule Compositions Stored for Three Months at 40°C (75% RH).***

| **Formulation** | **Weeks** | **Water By KF (%)** | **% assay Cmpd 1** | **% Area** | |
|---|---|---|---|---|---|
| | | | | **RRT 0.56** | **RRT 1.27** |
| Composition 13 (25 mg) | Initial | 4.3 | 98.9 | - | 0.16 |
| | 4 | 5.3 | 100.2 | - | 0.16 |
| | 8 | 4.1 | 100.6 | - | 0.16 |
| | 12 | 4.4 | 99.2 | - | 0.16 |
| Composition 13 (100 mg) | Initial | 4.2 | 98.1 | 0.05 | 0.16 |
| | 4 | 4.8 | 99.7 | - | 0.16 |
| | 8 | 3.7 | 97.7 | - | 0.16 |
| | 12 | 3.5 | 97.8 | - | 0.16 |
| Composition 14 (30 mg) | Initial | 4.4 | 99.2 | - | 0.14 |
| | .4 | 4.3 | 102.7 | - | 0.14 |
| | 8 | 3.8 | 99.3 | - | 0.14 |
| | 12 | 3.8 | 99.4 | - | 0.14 |
| Composition 14 (100 mg) | Initial | 4.2 | 104.6 | - | 0.14 |
| | 4 | 4.3 | 102.5 | - | 0.14 |
| | 8 | 4.2 | 101.7 | - | 0.14 |
| | 12 | 4.7 | 101.2 | - | 0.14 |
| Composition 15 (30 mg) | Initial | 3.6 | 100.7 | - | 0.15 |
| | 4 | 3.3 | 101.5 | - | 0.15 |
| | 8 | 4.1 | 101.6 | - | 0.15 |
| | 12 | 3.5 | 100.3 | - | 0.15 |
| Composition 15 (100 mg) | Initial | 3.6 | 99.8 | 0.05 | 0.14 |
| | 4 | 3.3 | 100.1 | - | 0.15 |
| | 8 | 3.2 | 99.3 | - | 0.15 |
| | 12 | 3.6 | 98.5 | - | 0.15 |

| | | | | | |
|---|---|---|---|---|---|
| *The packaging configuration was a HDPE bottle with rayon coil and heat-induction seal | | | | | |

**Table 8. Stability Data for Capsule Compositions Stored for Six Months at 40°C (75% RH).***

| **Formulation** | **Weeks** | **Water By KF (%)** | **% assay Cmpd 1** | **% Area** | |
|---|---|---|---|---|---|
| | | | | **RRT 0.56** | **RRT 1.27** |
| Composition 14 (30 mg) | Initial | 4.4 | 99.2 | - | 0.14 |
| | 4 | 4.5 | 102.9 | - | 0.14 |
| | 8 | 3.8 | 100.4 | - | 0.14 |
| | 12 | 4.1 | 99.9 | - | 0.14 |
| | 24 | 4.5 | 98.1 | - | 0.15 |
| Composition 14 (100 mg) | Initial | 4.2 | 104.6 | - | 0.14 |
| | 4 | 4.2 | 103.1 | - | 0.14 |
| | 8 | 3.9 | 102.2 | - | 0.14 |
| | 12 | 4.9 | 102.6 | - | 0.14 |
| | 24 | 4.3 | 102.3 | - | 0.15 |

| | | | | | |
|---|---|---|---|---|---|
| *The packaging configuration was a HDPE bottle without rayon coil and heat-induction seal. | | | | | |

**Table 9. Stability Data for Capsule Compositions Stored for Three Months at 40°C (75% RH).***

| **Formulation** | **Weeks** | **Water By KF (%)** | **% assay Cmpd 1** | **% Area** | |
|---|---|---|---|---|---|
| | | | | **RRT 0.56** | **RRT 1.27** |
| Composition 14 (30 mg) | Initial | 4.4 | 99.2 | - | 0.14 |
| | 4 | 4.3 | 102.7 | - | 0.14 |
| | 8 | 3.8 | 99.3 | - | 0.14 |
| | 12 | 3.8 | 99.4 | - | 0.14 |
| | 24 | 5.3 | 99.2 | - | 0.15 |
| Composition 14 (100 mg) | Initial | 4.2 | 104.6 | - | 0.14 |
| | 4 | 4.3 | 102.5 | - | 0.14 |
| | 8 | 4.2 | 101.7 | - | 0.14 |
| | 12 | 4.7 | 101.2 | - | 0.14 |
| | 24 | 5.0 | 101.4 | - | 0.15 |

| | | | | | |
|---|---|---|---|---|---|
| *The packaging configuration was a HDPE bottle with rayon coil and heat-induction seal | | | | | |

### Dissolution Stability

Dissolution stability of the pharmaceutical compositions 13, 14, and 15 was determined in simulated gastric fluid using a USP Apparatus II at 50 rpm. The compositions were tested at 100 mg and 25 or 30 mg API dosages. Each composition was found to be completely dissolved in the simulated gastric fluid after 60 minutes. The results of the stability studies are shown in Tables 10-15.

**Table 10. Dissolution stability of Composition 13 (25 mg) Capsule Composition Stored at 40°C (75% RH).**

| **Time (minutes)** | **Initial** | **1 month w/ rayon** | **2 months w/ rayon** | **3 months w/ rayon** | **1 month w/o rayon** | **2 months w/o rayon** | **3 months w/o rayon** |
|---|---|---|---|---|---|---|---|
| 10 | 80.9 | 89.7 | 81.0 | 85.9 | 81.3 | 78.9 | 69.6 |
| 20 | 88.1 | 99.9 | 94.1 | 92.0 | 92.0 | 90.9 | 85.8 |
| 30 | 94.1 | 105.5 | 97.6 | 95.3 | 98.3 | 95.1 | 92.2 |
| 45 | 96.5 | 108.6 | 99.7 | 97.9 | 102.5 | 98.9 | 97.0 |
| 60 | 99.4 | 110.0 | 100.6 | 99.2 | 102.3 | 100.5 | 99.2 |

**Table 11. Dissolution stability of Composition 13 (100 mg) Capsule Composition Stored at 40°C (75% RH).**

| **Time (minutes)** | **Initial** | **1 month w/ rayon** | **2 months w/ rayon** | **3 months w/ rayon** | **1 month w/o rayon** | **2 months w/o rayon** | **3 months w/o rayon** |
|---|---|---|---|---|---|---|---|
| 10 | 85.6 | 77.8 | 79.2 | 79.4 | 70.0 | 69.4 | 77.4 |
| 20 | 91.6 | 89.1 | 89.2 | 88.2 | 84.1 | 85.0 | 88.6 |
| 30 | 93.7 | 93.6 | 91.9 | 91.5 | 92.9 | 89.8 | 93.3 |
| 45 | 94.7 | 95.8 | 94.3 | 94.4 | 96.4 | 93.1 | 96.4 |
| 60 | 95.2 | 97.3 | 95.7 | 95.9 | 98.0 | 94.7 | 98.2 |

**Table 12. Dissolution stability of Composition 14 (30 mg) Capsule Composition Stored at 40°C (75% RH).**

| **Time (minutes)** | **Initial** | **1 month w/ rayon** | **2 months w/ rayon** | **3 months w/ rayon** | **6 months w/ rayon** |
|---|---|---|---|---|---|
| 10 | 92.0 | 94.0 | 87.2 | 97.3 | 96.3 |
| 20 | 97.4 | 101.1 | 95.0 | 100.6 | 102.3 |
| 30 | 98.6 | 104.7 | 98.4 | 102.3 | 104.1 |
| 45 | 99.6 | 106.6 | 100.4 | 103.5 | 105.1 |
| 60 | 100.1 | 105.7 | 100.6 | 103.9 | 105.8 |

| **Time (minutes)** | **Initial** | **1 month w/o rayon** | **2 months w/o rayon** | **3 months w/o rayon** | **6 months w/o rayon** |
|---|---|---|---|---|---|
| 10 | 92.0 | 99.6 | 89.9 | 89.4 | 97.4 |
| 20 | 97.4 | 103.8 | 97.4 | 97.4 | 101.7 |
| 30 | 98.6 | 104.2 | 99.4 | 99.9 | 103.4 |
| 45 | 99.6 | 105.7 | 101.1 | 101.5 | 104.4 |
| 60 | 100.1 | 105.1 | 101.7 | 102.1 | 104.8 |

**Table 13. Dissolution stability of Composition 14 (100 mg) Capsule Composition Stored at 40°C (75% RH).**

| **Time (minutes)** | **Initial** | **1 month w/ rayon** | **2 months w/ rayon** | **3 months w/ rayon** | **1 month w/o rayon** | **2 months w/o rayon** | **3 months w/o rayon** |
|---|---|---|---|---|---|---|---|
| 10 | 94.0 | 94.8 | 99.5 | 95.7 | 95.5 | 94.0 | 100.7 |
| 20 | 100.7 | 100.1 | 104.0 | 100.8 | 100.1 | 99.9 | 104.4 |
| 30 | 102.4 | 102.0 | 104.9 | 102.7 | 101.5 | 101.9 | 105.2 |
| 45 | 104.5 | 103.9 | 105.6 | 102.0 | 102.7 | 103.2 | 105.8 |
| 60 | 105.0 | 104.5 | 106.0 | 102.6 | 103.3 | 104.2 | 106.1 |

**Table 14. Dissolution stability of Composition 15 (30 mg) Capsule Composition Stored at 40°C (75% RH).**

| **Time (minutes)** | **Initial** | **1 month w/ rayon** | **2 months w/ rayon** | **3 months w/ rayon** | **1 month w/o rayon** | **2 months w/o rayon** | **3 months w/o rayon** |
|---|---|---|---|---|---|---|---|
| 10 | 89.4 | 73.2 | 75.7 | 62.7 | 74.0 | 76.0 | 76.3 |
| 20 | 98.3 | 97.3 | 95.0 | 89.8 | 94.5 | 96.1 | 93.9 |
| 30 | 100.3 | 101.3 | 99.5 | 95.7 | 101.2 | 99.3 | 99.3 |
| 45 | 101.6 | 103.6 | 101.4 | 98.7 | 104.4 | 101.3 | 101.8 |
| 60 | 101.4 | 107.2 | 102.2 | 100.4 | 106.7 | 102.2 | 102.8 |

**Table 15. Dissolution stability of Composition 15 (100 mg) Capsule Composition Stored at 40°C (75% RH).**

| **Time (minutes)** | **Initial** | **1 month w/ rayon** | **2 months w/ rayon** | **3 months w/ rayon** | **1 month w/o rayon** | **2 months w/o rayon** | **3 months w/o rayon** |
|---|---|---|---|---|---|---|---|
| 10 | 74.1 | 68.2 | 66.4 | 59.9 | 62.4 | 61.4 | 52.0 |
| 20 | 96.9 | 92.9 | 90.7 | 90.1 | 90.9 | 87.4 | 82.6 |
| 30 | 99.1 | 97.7 | 95.8 | 97.1 | 97.9 | 94.3 | 94.2 |
| 45 | 100.2 | 99.2 | 98.0 | 98.7 | 100 | 96.9 | 98.0 |
| 60 | 99.8 | 100.6 | 99.1 | 99.4 | 101 | 98.0 | 99.7 |

### Example 10

### Powder Analysis studies on 4-Amino-5-fluoro-3-[6-(4-methyl-piperazin-1-yl)-1H-benzimidazol-2-yl]-1H-quinolin-2-one formulations.

A drug loading study of API at 200 mg strength was undertaken. Formulations labeled Compositions 16, 17, 18, and 19 (Tables 16-19), at loadings of 70%, 60%, 50%, and 60%, respectively, were prepared using polyethylene bag blending techniques. The API and excipients, except for the magnesium stearate, were bag blended in a PE bag for 3 minutes. The mix was then passed through a number 30 mesh hand screen and charged into PE bags for 3 minutes of additional bag blending. The magnesium stearate was then passed through the number 30 mesh hand screen, added to the blend, and bag mixed for another three minutes or until the blend appeared uniform by visual inspection.

All compositions were evaluated at the 200 mg strength for acceptable flow properties. All flow testing was evaluated by Carr Index determination. The Carr Index was calculated according to the following formula: (tap density - bulk density)/(tap density). Table 20 shows the results of the evaluation including bulk density, tap density, Carr index and angle of repose. Each of the compositions shown in Tables 16-19 were found to have acceptable flow properties. Therefore, various embodiments include any of the compositions described herein.

**Table 16. Composition 16 in Capsule Size "0."**

| **Ingredient** | **Composition 16** | |
|---|---|---|
| | % **(w/w)** | **Mg/capsule** |
| Lactic acid salt of 4-amino-5-fluoro-3-[6-(4-methyl-piperazin-1-yl)-1H-benzimidazol-2-yl]-1H-quinolin-2-one | 70 | 246.00 |
| Partially Pregelatinized Starch | 13 | 45.69 |
| Cab-o-sil | 1 | 3.51 |
| Avicel PH 102 | 13 | 45.69 |
| Crospovidone | 2 | 7.03 |
| Magnesium stearate | 1 | 3.51 |
| Total | 100 | 351.43 |

**Table 17. Composition 17 in Capsule Size "0EL."**

| **Ingredient** | **Composition 17** | |
|---|---|---|
| | % **(w/w)** | **Mg/capsule** |
| Lactic acid salt of 4-amino-5-fluoro-3-[6-(4-methyl-piperazin-1-yl)-1H-benzimidazol-2-yl]-1H-quinolin-2-one | 60 | 249.00 |
| Partially Pregelatinized Starch | 18 | 74.70 |
| Cab-o-sil | 1 | 4.15 |
| Avicel PH 102 | 18 | 74.70 |
| Crospovidone | 2 | 8.30 |
| Magnesium stearate | 1 | 4.15 |
| Total | 100 | 415.00 |

**Table 18. Composition 18 in Capsule Size "0EL."**

| **Ingredient** | **Composition 18** | |
|---|---|---|
| | **% (w/w)** | **Mg/capsule** |
| Lactic acid salt of 4-amino-5-fluoro-3-[6-(4-methyl-piperazin-1-yl)-1H-benzimidazol-2-yl]-1H-quinolin-2-one | 50 | 246.00 |
| Partially Pregelatinized Starch | 23 | 113.16 |
| Cab-o-sil | 1 | 4.92 |
| Avicel PH 102 | 23 | 113.16 |
| Crospovidone | 2 | 9.84 |
| Magnesium stearate | 1 | 4.92 |
| Total | 100 | 492.00 |

**Table 19. Composition 19 in Capsule Size "0EL.**

| **Ingredient** | **Composition 19** | |
|---|---|---|
| | **% (w/w)** | **Mg/capsule** |
| Lactic acid salt of 4-amino-5-fluoro-3-[6-(4-methyl-piperazin-1-yl)-1H-benzimidazol-2-yl]-1H-quinolin-2-one | 60 | 246.00 |
| Partially Pregelatinized Starch | 30 | 123.00 |
| Cab-o-sil | 1 | 4.10 |
| Avicel PH 102 | 6 | 24.60 |
| Crospovidone | 2 | 8.20 |
| Magnesium stearate | 1 | 4.10 |
| Total | 100 | 410.00 |

**Table 20. Carr Index values for Compositions 16, 17, 18, and 19.**

| **Size or Property** | **Composition 16** | **Composition 17** | **Composition 18** | **Composition 19** |
|---|---|---|---|---|
| 40 Mesh | 5.9 | 4.4 | 2.3 | 5.1 |
| 60 Mesh | 21.8 | 18.3 | 11.4 | 18.3 |
| 80 Mesh | 8.7 | 8.7 | 7.8 | 9.4 |
| 120 Mesh | 12.9 | 13.9 | 13.7 | 12.6 |
| 200 Mesh | 20.4 | 21.9 | 24.9 | 25.6 |
| 325 Mesh | 18.0 | 19.4 | 21.8 | 16.5 |
| Pan | 12.3 | 13.5 | 18.0 | 12.5 |
| | | | | |
| Bulk Density (g/ml) | 0.48 | 0.54 | 0.51 | 0.52 |
| Tap Density (g/ml) | 0.60 | 0.64 | 0.62 | 0.67 |
| Carr's Index (5) | 20.0 | 15.6 | 12.9 | 22.4 |
| Angle of Repose (°) | 28.4 | 25.1 | N/A | 29.2 |

### Example 11

### Wet Granulated Formulations of 4-Amino-5-fluoro-3-[6-(4-methyl-piperazin-1-yl)-1H-benzimidazol-2-yl]-1H-quinolin-2-one

Capsule formulations were prepared in a manner similar to the general method of Example 10 with two additional steps: (1) The primary mixing is carried out in the presence of a granulating fluid such as aqueous, alcoholic, or hydro alcoholic fluids, and (2), a drying step is utilized to remove the granulating fluid. The lactic acid salt of 4-amino-5-fluoro-3-[6-(4-methyl-piperazin-1-yl)-1 H-benzimidazol-2-yl]-1 H-quinolin-2-one was prepared as described above and the anhydrous crystalline form was preferably used to prepare the formulations described herein (Form A). The various formulations were prepared on a scale of about 15 g and each capsule was targeted to contain about 15 mg of API. In addition to the lactic acid salt of 4-amino-5-fluoro-3-[6-(4-methyl-piperazin-1-yl)-1H-benzimidazol-2-yl]-1H-quinolin-2-one, lactose monohydrate, microcrystalline cellulose, and partially pregelatinized starch were added as diluents; povidone was added as a binder; crospovidone was added as a disintegrant; silicon dioxide was added as a flow aid; sodium lauryl sulphate was added as a wetting agent; and water or 0.5N HCl was added as the solvent or granulating fluid.

The procedure was as follows: all of the ingredients were combined and premixed dry (2 minutes with impeller at 650 rpm and chopper at 2500 rpm) except for the sodium lauryl sulfate and one half the crospovidone. The sodium lauryl sulfate, where present, was dissolved in the granulating fluid. While mixing at an impeller speed of 650 (chopper off), the granulating fluid was added by pipette to the mixture. After granulation liquid addition was completed, the chopper was turned on (2500 rpm) and the mixture was further mixed for 2 minutes. The wet granulation mixture was passed through a 20 mesh screen and the mixture dried in an oven at about 50°C until the loss on drying was less than 1%. The granulation was sized again with a 20 mesh screen, and further blended with the remaining crospovidone for 10 minutes.

The compositions were encapsulated to provide the 15 mg API in each capsule. The capsules used were either white opaque size #0 gelatin capsules from Capsugel or HPMC capsules from Shanogi. The same or a similar procedure may be used to prepare capsules other than the sizes described. For example, the same procedure may be used to prepare capsules of 25 mg, 30 mg, 50 mg, 100 mg, 150 mg, 200 mg, 250 mg, 300 mg, 350 mg, 400 mg, 450 mg, and 500 mg by simply adjusting the appropriate capsule size and the amount of the ingredients in the composition as will be apparent to those of skill in the art.

Table 21 shows 12 compositions (20-31) of wet formulations produced by the above-described methods.

**Table 21. Wet Granulated Compositions**

| **Comp. ID No.** | **HC** | **Capsule type** | **(% w/w)** | | | | | | | |
|---|---|---|---|---|---|---|---|---|---|---|
| | | | **PS** | **CP** | **SLS** | **PD** | **SiO₂** | **Lactose** | **MCC** | **API** |
| 20 | 1 | G | 0 | 5 | 0 | 0 | 0 | 35 | 0 | 60 |
| 21 | -1 | G | 0 | 2 | 0 | 4 | 1 | 35 | 35 | 23 |
| 22 | 1 | H | 10 | 5 | 0 | 4 | 1 | 0 | 0 | 80 |
| 23 | -1 | G | 0 | 2 | 0 | 0 | 0 | 0 | 0 | 98 |
| 24 | 1 | G | 0 | 5 | 1 | 4 | 0 | 35 | 35 | 20 |
| 25 | 1 | G | 10 | 2 | 0 | 0 | 1 | 35 | 35 | 17 |
| 26 | 1 | H | 10 | 2 | 1 | 0 | 0 | 0 | 35 | 52 |
| 27 | -1 | H | 10 | 5 | 0 | 4 | 0 | 0 | 35 | 46 |
| 28 | 1 | H | 0 | 2 | 1 | 4 | 1 | 0 | 0 | 92 |
| 29 | -1 | H | 10 | 5 | 1 | 0 | 1 | 35 | 0 | 48 |
| 30 | -1 | G | 10 | 2 | 1 | 4 | 0 | 35 | 0 | 48 |
| 31 | -1 | G | 0 | 5 | 1 | 0 | 1 | 0 | 35 | 58 |

| | | | | | | | | | | |
|---|---|---|---|---|---|---|---|---|---|---|
| HC = granulating liquid: 1 = water as granulating liquid; -1 = 0.5 N HCl as granulating liquid. G = Hard Gelatin capsule; H = Hydroxymethylpropyl cellulose capsule PS = Partially Pregelatinized Starch CP = Crospovidone PD = Povidone SLS = Sodium lauryl sulfate MCC = Microcrystalline cellulose API = Compound of formula I | | | | | | | | | | |

### Example 12

### Formulation Evaluation in Dogs

Four formulations were evaluated in dogs. The formulations included Composition 13, Composition 14, Composition 15 and a powder-in-bottle (PIB) formulation. The dosage administered to the dogs was 100 mg of the compound per dog per period (100 mg/dog/period). The number of animals in the study was four male dogs and four female dogs (N = 4/sex). A four-way randomized crossover study design was used in the evaluation. A 1-week washout was used between treatments. The lower limit of quantification (LLOQ) was about 1 ng/mL. AUC (ng*hr/mL) of each composition was evaluated and found to range from 100 to 350 ng*hr/mL. The PIB composition produced AUCs ranging from 150-450 ng*hr/mL. No apparent gender effects were observed. No treatment or period effects were observed. Similar observations were made with respect to Cₘₐₓ. These studies showed that each of the compositions, and particularly composition 14, has desirable properties as a pharmaceutical formulation.

The contents of each of the patents, patent applications and journal articles cited above are hereby incorporated by reference herein and for all purposes as if fully set forth in their entireties.

It is understood that the invention is not limited to the embodiments set forth herein for illustration, but embraces all such forms thereof as come within the scope of the disclosure presented herein. The present invention further provides the following enumerated embodiments.

### Enumerated Embodiments

1. A pharmaceutical formulation, comprising:
   a compound of formula I, a tautomer of the compound, a pharmaceutically acceptable salt of the compound, a pharmaceutically acceptable salt of the tautomer, or a mixture thereof, 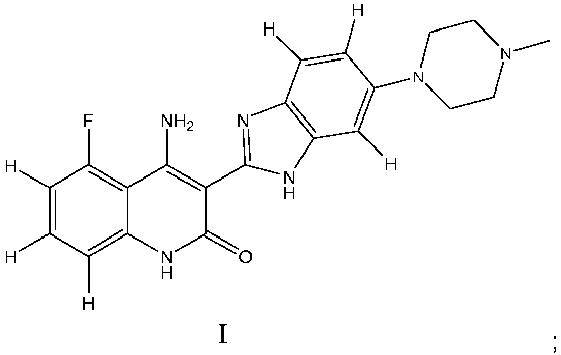 and
   at least one ingredient selected from the group consisting of (i) cellulose; (ii) lactose, starch, or a mixture thereof; (iii) povidone; (iv) silicon dioxide or talc; (v) a pharmaceutically acceptable lubricant; and (vi) an ingredient selected from crospovidone, croscarmellose sodium; or sodium starch glycolate.
2. The pharmaceutical formulation of enumerated embodiment 1, wherein the pharmaceutically acceptable lubricant is selected from the group consisting of a C₁₆₋₂₂ fatty acid, a salt of a C₁₆₋₂₂ fatty acid, a C₁₆₋₂₂ fatty acid ester, a salt of a C₁₆₋₂₂ fatty acid ester; a polyethylene glycol having an average molecular weight of 6,000 to 10,000.
3. The pharmaceutical formulation of enumerated embodiment 1, wherein the formulation comprises:
   (i) cellulose;
   (ii) silicon dioxide;
   (iii) stearic acid or a salt of stearic acid; and
   (iv) at least one ingredient selected from crospovidone, starch, lactose, croscarmellose sodium, or sodium starch glycolate.
4. The pharmaceutical formulation of any one of enumerated embodiments 1-3, wherein the formulation comprises a lactic acid salt of the compound of formula I.
5. The formulation of any of enumerated embodiments 1-4, wherein the lactic acid salt of the compound of formula I is in an anhydrous crystalline form.
6. The formulation of enumerated embodiment 5 wherein the anhydrous crystalline form is Form A.
7. The pharmaceutical formulation of enumerated embodiment 1, wherein the formulation comprises:
   (i) microcrystalline cellulose;
   (ii) silicon dioxide;
   (iii) magnesium stearate;
   (iv) at least one ingredient selected from crospovidone, partially pregelatinized starch, and lactose.
8. The pharmaceutical formulation of any one of enumerated embodiments 1-7, wherein the formulation comprises the lactic acid salt of the compound in an amount ranging from 10% to 50% by weight based on the total weight of the formulation.
9. The pharmaceutical formulation of enumerated embodiment 8, wherein the formulation comprises the lactic acid salt of the compound in an amount ranging from 20% to 45% by weight based on the total weight of the formulation.
10. The pharmaceutical formulation of enumerated embodiment 8, wherein the formulation comprises the lactic acid salt of the compound in an amount ranging from 30% to 40% by weight based on the total weight of the formulation.
11. The pharmaceutical formulation of any one of enumerated embodiments 1-10, wherein the cellulose is microcrystalline cellulose.
12. The pharmaceutical formulation of any one of enumerated embodiments 1-11, wherein the formulation comprises the cellulose in an amount ranging from 10% to 70% by weight based on the total weight of the formulation.
13. The pharmaceutical formulation of enumerated embodiment 12, wherein the formulation comprises the cellulose in an amount ranging from 20% to 50% by weight based on the total weight of the formulation, and the formulation comprises crospovidone in an amount ranging from 2% to 6% by weight based on the total weight of the formulation.
14. The pharmaceutical formulation of any one of enumerated embodiments 1-11, wherein the formulation comprises the cellulose in an amount ranging from 20% to 45% by weight based on the total weight of the formulation, and the formulation comprises starch or lactose in an amount ranging from 10% to 40% by weight based on the total weight of the formulation.
15. The pharmaceutical formulation of enumerated embodiment 14, wherein the formulation comprises the starch in an amount ranging from 10% to 40% by weight based on the total weight of the formulation, and the starch is partially pregelatinized starch.
16. The pharmaceutical formulation of any one of enumerated embodiments 1-15, wherein the formulation comprises the silicon dioxide in an amount ranging from 0.3% to 2% by weight based on the total weight of the formulation.
17. The pharmaceutical formulation of any one of enumerated embodiments 1-16, wherein the formulation comprises magnesium stearate in an amount ranging from 0.1% to 2% by weight based on the total weight of the formulation.
18. The pharmaceutical formulation of any one of enumerated embodiments 1-7, wherein the formulation comprises the lactic acid salt of the compound in an amount ranging from 30% to 40% by weight based on the total weight of the formulation; the silicon dioxide in an amount ranging from 0.3% to 2% by weight based on the total weight of the formulation, the cellulose in an amount ranging from 25% to 40% of the total weight of the formulation, magnesium stearate in an amount ranging from 0.1 % to 2% by weight based on the total weight of the formulation, and the crospovidone in an amount ranging from 2% to 4% by weight based on the total weight of the formulation.
19. The pharmaceutical formulation of any one of enumerated embodiments 1-7, wherein the formulation comprises the lactic acid salt of the compound in an amount ranging from 50% to 80% by weight based on the total weight of the formulation; the silicon dioxide in an amount ranging from 0.3% to 2% by weight based on the total weight of the formulation, the cellulose in an amount ranging from 0% to 50% of the total weight of the formulation, magnesium stearate in an amount ranging from 0.1 % to 2% by weight based on the total weight of the formulation, and the starch in an amount ranging from 10% to 40% by weight based on the total weight of the formulation.
20. The pharmaceutical formulation of enumerated embodiment 19, wherein the formulation comprises the lactic acid salt of the compound in an amount ranging from 55% to 75% by weight based on the total weight of the formulation, the cellulose in an amount ranging from 5% to 40% of the total weight of the formulation, and the starch in an amount ranging from 15% to 30% by weight based on the total weight of the formulation.
21. The pharmaceutical formulation of enumerated embodiment 19, wherein the formulation comprises the lactic acid salt of the compound in an amount ranging from 60% to 70% by weight based on the total weight of the formulation and the cellulose in an amount ranging from 5% to 25% of the total weight of the formulation.
22. The pharmaceutical formulation of any one of enumerated embodiments 1-7, wherein the formulation comprises the lactic acid salt of the compound in an amount ranging from 50% to 80% by weight based on the total weight of the formulation; the silicon dioxide in an amount ranging from 0.3% to 2% by weight based on the total weight of the formulation, the cellulose in an amount ranging from 0% to 50% of the total weight of the formulation, magnesium stearate in an amount ranging from 0.1 % to 2% by weight based on the total weight of the formulation, and the lactose in an amount ranging from 10% to 40% by weight based on the total weight of the formulation.
23. The pharmaceutical formulation of enumerated embodiment 22, wherein the formulation comprises the lactic acid salt of the compound in an amount ranging from 55% to 75% by weight based on the total weight of the formulation and the cellulose in an amount ranging from 5% to 40% of the total weight of the formulation.
24. The pharmaceutical formulation of enumerated embodiment 22, wherein the formulation comprises the lactic acid salt of the compound in an amount ranging from 60% to 70% by weight based on the total weight of the formulation and the cellulose in an amount ranging from 5% to 40% of the total weight of the formulation.
25. A pharmaceutical formulation, comprising:
   a compound of formula I, a tautomer of the compound, a pharmaceutically acceptable salt of the compound, a pharmaceutically acceptable salt of the tautomer, or a mixture thereof, 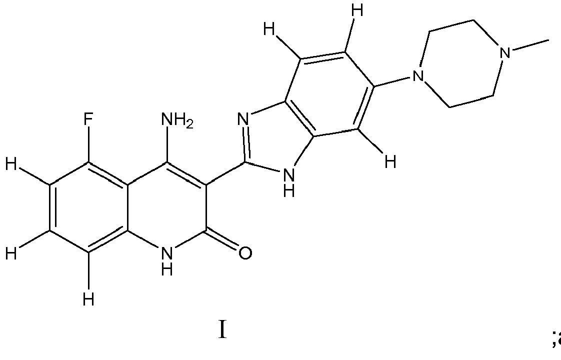 and
      at least one ingredient selected from the group consisting of cellulose, povidone, silicon dioxide, talc, and a pharmaceutically acceptable lubricant; and at least one ingredient selected from the group consisting of lactose, starch, crospovidone, croscarmellose sodium, and sodium starch glycolate.
26. The pharmaceutical formulation of any one of enumerated embodiments 1-25 further comprising an antioxidant, a chelating agent, ascorbic acid, a reducing sugar, or a mixture of any two or more thereof.
27. The pharmaceutical formulation of any one of enumerated embodiments 1-26, wherein the formulation is contained within a capsule.
28. The pharmaceutical formulation of any one of enumerated embodiments 1-26, wherein the formulation is contained within a tablet.
29. The pharmaceutical formulation of enumerated embodiment 27 or enumerated embodiment 28, wherein the total mass of the compound of formula I, the tautomer of the compound, the lactic acid salt of the compound, the lactic acid salt of the tautomer, or the mixture thereof in the capsule or tablet ranges from 25 mg to 500 mg.
30. The pharmaceutical formulation of any one of enumerated embodiments 1-27, wherein the amount of degradants of the compound of formula I is less than 10% by weight based on the total weight of the formulation after storage of the formulation for three months at 40°C and 75% room humidity.
31. A method for producing a pharmaceutical formulation, comprising:
   (a) blending a first mixture to provide a first blended mixture, the first mixture comprising:
      (i) a compound of formula I, a tautomer of the compound, a pharmaceutically acceptable salt of the compound, a pharmaceutically acceptable salt of the tautomer, or a mixture thereof, and 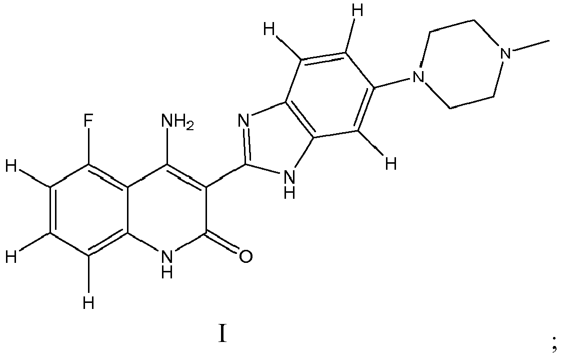
      (ii) at least one ingredient selected from the group consisting of cellulose; lactose, starch, or a mixture thereof; povidone; silicon dioxide or talc; a pharmaceutically acceptable lubricant; and an ingredient selected from crospovidone, croscarmellose sodium; or sodium starch glycolate.
32. The method of enumerated embodiment 31, wherein the compound of formula I is blended with (i) cellulose; (ii) silicon dioxide; and (iii) an ingredient selected from crospovidone, starch, or lactose.
33. The method of enumerated embodiment 32 further comprising
   (b) blending stearic acid, a salt of stearic acid, or a mixture thereof with the first blended mixture to provide a second blended mixture.
34. The method of enumerated embodiment 33 further comprising (c) forming at least one capsule or at least one tablet from the second blended mixture.
35. A method for producing a pharmaceutical formulation, comprising:
   (a) blending a mixture of ingredients to provide a first blended mixture, the blended mixture comprising:
      (i) a compound of formula I, a tautomer of the compound, a pharmaceutically acceptable salt of the compound, a pharmaceutically acceptable salt of the tautomer, or a mixture thereof, 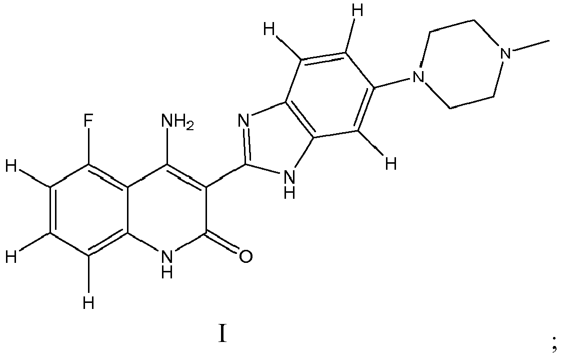
      (ii) at least one ingredient selected from the group consisting of cellulose; starch; lactose; and povidone;
      (iii) at least one ingredient selected from the consisting of crospovidone; croscarmellose sodium; and sodium starch glycolate;
      (iv) a granulating fluid selected from the group consisting of aqueous acid; alcohol; aqueous alcohol, or a mixture of any two or more thereof;
   (b) removing the granulating fluid;
   (c) producing a second blended mixture by blending the first blended mixture with at least one additional ingredient selected from the group consisting of:
      (i) crospovidone, croscarmellose sodium, or sodium starch glycolate;
      (ii) stearic acid or a salt of stearic acid; and
      (iii) silicon dioxide or talc.
36. The method of enumerated embodiment 35 further comprising (d) forming at least one capsule or at least one tablet from the second blended mixture.
37. The method of enumerated embodiments 34 or 36, wherein the total mass of the compound of formula I, the tautomer of the compound, the pharmaceutically acceptable salt of the compound, the pharmaceutically acceptable salt of the tautomer, or the mixture thereof in the capsule or tablet ranges from 25 mg to 500 mg.
38. The method of enumerated embodiment 35 wherein steps (a), (b), and (c) are performed simultaneously.
39. The method of any one of enumerated embodiments 33-36, wherein the second blended mixture comprises a lactic acid salt of the compound of formula I.
40. The method of enumerated embodiment 39, wherein the second blended mixture comprises the lactic acid salt of the compound in an amount ranging from 10% to 50% by weight based on the total weight of the second blended mixture.
41. The method of enumerated embodiment 39, wherein the second blended mixture comprises the lactic acid salt of the compound in an amount ranging from 20% to 45% by weight based on the total weight of the second blended mixture.
42. The method of enumerated embodiment 39, wherein the second blended mixture comprises the lactic acid salt of the compound in an amount ranging from 30% to 40% by weight based on the total weight of the second blended mixture.
43. The method of any one of enumerated embodiments 33-42, wherein the second blended mixture comprises the cellulose in an amount ranging from 10% to 70% by weight based on the total weight of the second blended mixture.
44. The method of enumerated embodiment 43, wherein the second blended mixture comprises the cellulose in an amount ranging from 20% to 50% by weight based on the total weight of the second blended mixture, and the second blended mixture comprises crospovidone in an amount ranging from 2% to 6% by weight based on the total weight of the second blended mixture.
45. The method of any one of enumerated embodiments 33-43, wherein the second blended mixture comprises the cellulose in an amount ranging from 20% to 50% by weight based on the total weight of the second blended mixture, and the second blended mixture comprises starch or lactose in an amount ranging from 10% to 40% by weight based on the total weight of the second blended mixture.
46. The method of any one of enumerated embodiments 31-45, wherein the cellulose is microcrystalline cellulose.
47. The method of any one of enumerated embodiments 33-43, wherein the second blended mixture comprises the starch in an amount ranging from 20% to 40% by weight based on the total weight of the second blended mixture, and the starch is partially pregelatinized starch.
48. The method of any one of enumerated embodiments 33-43, wherein the second blended mixture comprises the silicon dioxide in an amount ranging from 0.3% to 2% by weight based on the total weight of the second blended mixture.
49. The method of any one of enumerated embodiments 33-43, wherein the second blended mixture comprises the magnesium stearate in an amount ranging from 0.1 % to 2% by weight based on the total weight of the second blended mixture.
50. The method of any one of enumerated embodiments 33-38, wherein the second blended mixture comprises a lactic acid salt of the compound in an amount ranging from 50% to 80% by weight based on the total weight of the second blended mixture.
51. The method of enumerated embodiment 50 wherein the second blended mixture comprises a lactic acid salt of the compound in an amount ranging from 55% to 75% by weight based on the total weight of the second blended mixture.
52. The method of enumerated embodiment 50 wherein the second blended mixture comprises a lactic acid salt of the compound in an amount ranging from 60% to 70% by weight based on the total weight of the second blended mixture.
53. The method of any one of enumerated embodiments 50-52, wherein the second blended mixture further comprises silicon dioxide in an amount ranging from 0.3% to 2% by weight based on the total weight of the second blended mixture.
54. The method of any one of enumerated embodiments 50-52, wherein the second blended mixture further comprises the cellulose in an amount ranging from 20% to 45% of the total weight of the second blended mixture.
55. The method of any one of enumerated embodiments 50-52, wherein the second blended mixture further comprises the magnesium stearate in an amount ranging from 0.1 % to 2% by weight based on the total weight of the second blended mixture.
56. The method of any one of enumerated embodiments 50-52, wherein the second blended mixture further comprises the crospovidone in an amount ranging from 2% to 6% by weight based on the total weight of the second blended mixture.
57. The method of any one of enumerated embodiments 50-52, wherein the second blended mixture comprises silicon dioxide in an amount ranging from 0.5% to 2% by weight based on the total weight of the second blended mixture, the cellulose in an amount ranging from 20% to 45% of the total weight of the second blended mixture, the magnesium stearate in an amount ranging from 0.5% to 2% by weight based on the total weight of the second blended mixture, and the crospovidone in an amount ranging from 2% to 4% by weight based on the total weight of the second blended mixture.
58. A pharmaceutical packaging container, comprising: a storage vessel comprising two or more capsules or tablets, the capsules or tablets comprising the pharmaceutical formulation of any one of enumerated embodiments 1-30.
59. The pharmaceutical packaging container of enumerated embodiment 58, wherein the storage vessel comprises high density polyethylene.
60. The pharmaceutical packaging container of enumerated embodiment 59, wherein the storage vessel further comprises a cotton or rayon coil.
61. The pharmaceutical packaging container of enumerated embodiment 59, wherein the storage vessel further comprises a heat induction seal.
62. A pharmaceutical packaging container, comprising a blister package, the blister package comprising at least one capsule or tablet comprising the pharmaceutical formulation of any one of enumerated embodiments 1-30.
63. The pharmaceutical formulation of any one of enumerated embodiments 29, 34, or 36, wherein the tablet is coated with a substance selected from the group consisting of sugar, cellulose polymer, and polymethacrylate polymer.
64. The pharmaceutical formulation of any one of enumerated embodiments 29, 34, or 36, wherein the tablet is further coated with gelatin or encapsulated within a gelatin sheath.
65. The pharmaceutical formulation of enumerated embodiments 63-64, wherein the coating material further contains a pharmaceutically acceptable coloring agent.
66. The pharmaceutical formulation of enumerated embodiments 63-65, wherein the coating material further contains a pharmaceutically acceptable opacifier.
67. The method of any one of enumerated embodiments 31-57, where in the pharmaceutical formulation is manufactured using at least one apparatus selected from the group consisting of (i) a fluidized bed granulator equipped with a bottom spray, a top spray, or a tangential spray mechanism; (ii) a high shear granulator; (iii) a low shear granulator; (iv) a roller compactor; and (V) a tablet press.
68. A method for treating cancer and/or inhibiting angiogenesis in a subject, comprising: administering the formulation according to any one of enumerated embodiments 1-30 to the subject.
69. The method of enumerated embodiment 68, wherein the formulation is administered in an amount sufficient to provide a Cₘₐₓ of about 20 to 4000 ng/mL of the compound of formula I, the tautomer of the compound, the lactic acid salt of the compound, the lactic acid salt of the tautomer, or the mixture thereof in the subject's plasma or a Cₘₐₓ of about 40 to 8000 ng/mL of the compound of formula I, the tautomer of the compound, the lactic acid salt of the compound, the lactic acid salt of the tautomer, or the mixture thereof in the subject's blood.
70. The method of enumerated embodiment 68, wherein the formulation is administered in an amount sufficient to provide about 10 to 2,000 ng/mL of the compound of formula I, the tautomer of the compound, the lactic acid salt of the compound, the lactic acid salt of the tautomer, or the mixture thereof in the subject's plasma 24 hours after administration or about 20 to 4,000 ng/mL of the compound of formula I, the tautomer of the compound, the lactic acid salt of the compound, the lactic acid salt of the tautomer, or the mixture thereof in the subject's blood 24 hours after administration.
71. The method of enumerated embodiment 68, wherein the formulation is administered in an amount sufficient to provide to provide an AUC of about 500 to 60,000 ng*h/mL of the compound of formula I, the tautomer of the compound, the lactic acid salt of the compound, the lactic acid salt of the tautomer, or the mixture thereof in the subject's plasma or about 750 to 120,000 ng*h/mL of the compound of formula I, the tautomer of the compound, the lactic acid salt of the compound, the lactic acid salt of the tautomer, or the mixture thereof in the subject's blood.
72. The method of enumerated embodiment 68, wherein the formulation is administered once, twice, three times, or four times daily.
73. The method of enumerated embodiment 68, wherein the amount of the compound of formula I, the tautomer of the compound, the lactic acid salt of the compound, the lactic acid salt of the tautomer, or the mixture thereof administered to the subject ranges from 0.25 to 30 mg/kg body weight of the subject.
74. The method of enumerated embodiment 68, wherein the cancer to be treated is selected from prostate, colorectal, breast, multiple myeloma, pancreatic, small cell carcinoma, acute myelogenous leukemia, chronic myelogenous leukemia, myelo-proliferative disease, nonsmall cell lung, small cell lung, chronic lymphoid leukemia, sarcoma, melanoma, lymphoma, thyroid, neuroendocrine, renal cell, gastric, gastrointestinal stromal, glioma, brain, or bladder cancer.
75. The method of enumerated embodiment 68, further comprising administering the formulation as part of a treatment cycle, wherein the treatment cycle comprises administering the formulation daily for 7, 14, 21, or 28 days, followed by 7 or 14 days without administration of the formulation.
76. The method of enumerated embodiment 68, wherein the treatment cycle comprises administering the amount of the compound daily for 7 days, followed by 7 days without administration of the compound.
77. The method of enumerated embodiment 75 or enumerated embodiment 76, wherein the treatment cycle is repeated one or more times.

## Claims

1. A pharmaceutical formulation, comprising:
A lactic acid salt of the compound of formula I or a tautomer of the compound, or a mixture thereof, in an amount ranging from 50% to 80% by weight based on the total formulation 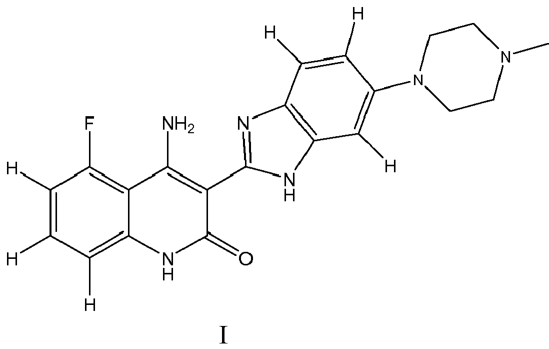 and
at least one ingredient selected from the group consisting of (i) cellulose; (ii) lactose, starch, or a mixture thereof; (iii) povidone; (iv) silicon dioxide or talc; (v) a pharmaceutically acceptable lubricant; and (vi) an ingredient selected from crospovidone, croscarmellose sodium; or sodium starch glycolate.

2. The pharmaceutical formulation of claim 1, wherein the pharmaceutically acceptable lubricant is selected from the group consisting of a C₁₆₋₂₂ fatty acid, a salt of a C₁₆₋₂₂ fatty acid, a C₁₆₋₂₂ fatty acid ester, a salt of a C₁₆₋₂₂ fatty acid ester; a polyethylene glycol having an average molecular weight of 6,000 to 10,000.

3. The formulation of any of claims 1-2, wherein the lactic acid salt of the compound of formula I is in an anhydrous crystalline form.

4. The formulation of claim 3, wherein the anhydrous crystalline form is Form A.

5. The pharmaceutical formulation of claim 1, wherein the formulation comprises:
(i) microcrystalline cellulose;
(ii) silicon dioxide;
(iii) magnesium stearate;
(iv) at least one ingredient selected from crospovidone, partially pregelatinized starch, and lactose.

6. The pharmaceutical formulation of claims 1-4, wherein the cellulose is microcrystalline cellulose.

7. The pharmaceutical formulation of any one of claims 1-6, wherein the formulation comprises the cellulose in an amount ranging from 20% to 50% by weight based on the total weight of the formulation, and the formulation comprises crospovidone in an amount ranging from 2% to 6% by weight based on the total weight of the formulation.

8. The pharmaceutical formulation of any one of claims 1-6, wherein the formulation comprises the cellulose in an amount ranging from 20% to 45% by weight based on the total weight of the formulation, and the formulation comprises starch or lactose in an amount ranging from 10% to 40% by weight based on the total weight of the formulation.

9. The pharmaceutical formulation of any one of claims 1-6, wherein the formulation comprises the starch in an amount ranging from 10% to 40% by weight based on the total weight of the formulation, and the starch is partially pregelatinized starch.

10. The pharmaceutical formulation of any one of claims 1-6, wherein the formulation comprises the lactic acid salt of the compound in an amount ranging from 50% to 80% by weight based on the total weight of the formulation; the silicon dioxide in an amount ranging from 0.3% to 2% by weight based on the total weight of the formulation, the cellulose in an amount ranging from 0% to 50% of the total weight of the formulation, magnesium stearate in an amount ranging from 0.1 % to 2% by weight based on the total weight of the formulation, and starch or the lactose in an amount ranging from 10% to 40% by weight based on the total weight of the formulation.

11. The pharmaceutical formulation of claim 10, wherein the formulation comprises the lactic acid salt of the compound in an amount ranging from 55% to 75% by weight based on the total weight of the formulation, the cellulose in an amount ranging from 5% to 40% of the total weight of the formulation, and the starch or lactose in an amount ranging from 15% to 30% by weight based on the total weight of the formulation.

12. The pharmaceutical formulation of claim 10, wherein the formulation comprises the lactic acid salt of the compound in an amount ranging from 55% to 75% by weight based on the total weight of the formulation and the cellulose in an amount ranging from 5% to 40% of the total weight of the formulation.

13. The pharmaceutical formulation of claim 10, wherein the formulation comprises the lactic acid salt of the compound in an amount ranging from 60% to 70% by weight based on the total weight of the formulation and the cellulose in an amount ranging from 5% to 40% of the total weight of the formulation.

14. The pharmaceutical formulation of claim 10, wherein the formulation comprises the lactic acid salt of the compound in an amount ranging from 60% to 70% by weight based on the total weight of the formulation and the cellulose in an amount ranging from 5% to 25% of the total weight of the formulation.

15. The pharmaceutical formulation of any one of claims 1-14 for use in a method of treating cancer and/or inhibiting angiogenesis in a subject.
